# EUROPÄISCHE PATENTSCHRIFT

(11) **EP 1 902 012 B1**
(45) Veröffentlichungstag und Bekanntmachung des Hinweises auf die Patenterteilung: **09.12.2015**
(21) Anmeldenummer: 06777361.4
(22) Anmeldetag: 19.06.2006
(51) Int. Cl.: C07C 209/62, C07C 209/88, C07B 53/00, C07C 213/02, C07C 213/10

(54) **VERFAHREN ZUR HERSTELLUNG VON OPTISCH AKTIVEN AMINOALKYLPHENOLEN**
METHOD FOR PRODUCING OPTICALLY ACTIVE AMINOALKYLPHENOLS
PROCÉDÉ DE FABRICATION D'AMINOALKYLPHENOLS OPTIQUEMENT ACTIFS

(30) Priorität: 20.06.2005 DE 102005028492
(43) Veröffentlichungstag der Anmeldung: 26.03.2008
(73) Patentinhaber: BASF SE, 67056 Ludwigshafen (DE)
(72) Erfinder: DITRICH, Klaus, 67161 Gönnheim (DE)
(86) Internationale Anmeldenummer: PCT/EP2006/063314
(87) Internationale Veröffentlichungsnummer: WO 2006/136538

(56) Entgegenhaltungen:
- EP-A- 0 311 385
- EP-A- 0 865 500
- WO-A-95/08636
- WO-A-96/23894
- DE-A1- 19 603 575
- US-A- 5 965 032
- US-B1- 6 576 795
- E.PETER KÜNDIG ET AL: "Asymmetric Syntheses of 2-(1-Aminoethyl)phenols" HELVETICA CHIMICA ACTA, Bd. 87, Nr. 3, 2004, Seiten 561-579, XP002394554

## Beschreibung

Die vorliegende Erfindung betrifft ein Verfahren zur enantioselektiven N-Acylierung von Aminoalkylphenolen sowie ein Verfahren zur Herstellung von enantiomerenreinen Verbindungen der Formeln (I-S) und/oder (I-R)

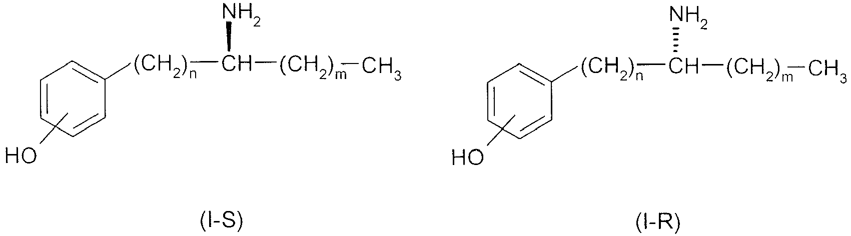

Die Herstellung von enantiomerenreinen Aminoalkylbenzolen durch Racematspaltung ist bekannt. So beschreibt die WO 95/08636 ein Verfahren zur Racematspaltung von primären und sekundären Aminen durch Umsetzung mit einem Ester in Gegenwart einer Hydrolase und anschließende Trennung des einen enantioselektiv acylierten Amins vom nicht acylierten Antipoden. Eine Racemattrennung von Alkylaminohydroxybenzolen wird hingegen nicht beschrieben.

Die US 4,904,680 beschreibt ein Verfahren zur Racematspaltung von 4-(1-Aminoethyl)phenol durch Umsetzung des Enantiomerengemischs mit Äpfelsäure. Die Umsetzung verläuft allerdings mit schlechten Ausbeuten und auch die optische Reinheit ist noch nicht zufriedenstellend.

Weitere Verfahren zur Racematspaltung von Aminen werden in EP 0 865 500 A, US 5 576 795 B1, DE 196 03 575 und US 5 965 032 A beschrieben. Dabei werden u. a. racemische Alkylaminobenzole, u. a. Alkoxy-substituierte Alkylaminobenzole, mit einem Ester in Gegenwart von Hydrolasen umgesetzt und anschließend aufgetrennt. Die Racematspaltung von Aminoalkylphenolen wird nicht beschrieben. Auch eine Hydrolyse von Alkoxy-substituierten Alkylaminobenzolen zu den entsprechenden Phenolen wird nicht erwähnt.

Die WO 96/23894 beschreibt die Racematspaltung strukturell verschiedener primärer Amine durch Umsetzung mit einem Ester in Gegenwart einer Hydrolase mit anschließender Trennung des enantioselektiv acylierten Amins vom nicht umgesetzten Amin.

In EP 0 311 385 A und in Helvetica Chimia Acta, 87(3), 2004, 561-579 werden Herstellungsverfahren von Aminophenolen beschrieben, die einen enantioselektiven Reduktionsschritt umfassen. Die beschriebenen Verfahren sind komplex und erfordern den Einsatz teurerer Reagenzien.

Aufgabe der vorliegenden Erfindung war es, ein effizientes Verfahren zur Herstellung von im Wesentlichen enantiomerenreinen Verbindungen der Formeln (I-S) und/oder (I-R) bereitzustellen.

Es wurde überraschenderweise gefunden, dass man Amine (I-S) und/oder (I-R) gewinnen kann, wenn man das Enantiomerengemisch, insbesondere das Racemat dieser Amine in Gegenwart einer Hydrolase enantioselektiv acyliert. Voraussetzung dabei ist, dass die Hydroxyfunktion am Benzolring vor der Acylierung geschützt wird.

Die Erfindung betrifft ein Verfahren (Verfahren B) zur Herstellung von im Wesentlichen enantiomerenreinen Verbindungen der Formeln (I-S) und/oder (I-R) 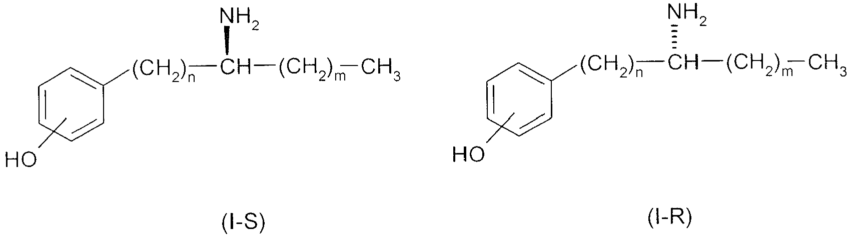 worin
- n: für 0, 1 oder 2 steht und
- m: für 0, 1 oder 2 steht,
umfassend die folgenden Schritte:
(i) Umsetzung eines Enantiomerengemischs der Formel (II) 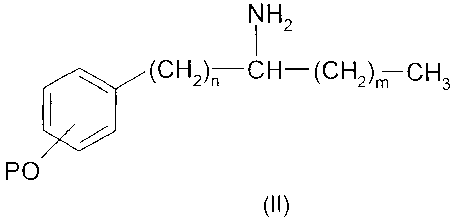 worin
   - P: für eine Schutzgruppe steht, die ausgewählt ist unter tert-Butyl und einer am Phenylring gegebenenfalls substituierten Benzylgruppe, und
   - m und n: wie vorstehend definiert sind,
   mit einem Acylierungsmittel in Gegenwart einer Hydrolase, wobei man ein Gemisch erhält, in welchem ein Enantiomer im Wesentlichen in der acylierten Form vorliegt und das andere Enantiomer im Wesentlichen in der nicht acylierten Form vorliegt;
(ii) Abtrennung des nicht acylierten Enantiomers der Verbindung (II) aus dem in Schritt (i) erhaltenen Gemisch;
(iii) Entschützen des in Schritt (ii) gewonnenen nicht acylierten Enantiomers der Verbindung (II) zum Amin (I-S) oder (I-R); und
(iv) gewünschtenfalls Hydrolyse des in Schritt (i) erhaltenen im Wesentlichen acylierten Enantiomers der Verbindung (II) zum entsprechenden nicht acylierten Enantiomer des Amins (II) und anschließendes Entschützen zum Amin (I-R) oder (I-S),
wobei die Verbindung der Formel (II) dadurch erhalten wird, dass man
(a) eine Verbindung der Formel (III) 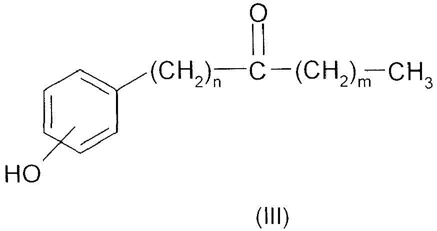 unter Erhalt der Verbindung (IV) an der OH-Gruppe schützt 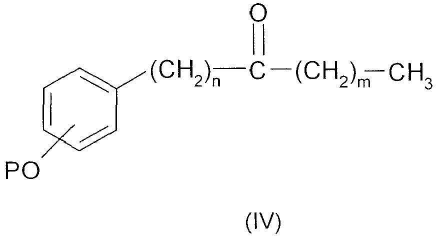 und
(b) die Verbindung (IV) einer reduktiven Aminierung unterwirft, wobei man die Verbindung der Formel (II) erhält.

Die nachfolgend gemachten Ausführungen zu bevorzugten Ausgestaltungen des erfindungsgemäßen Verfahrens, der Reaktanden und der Produkte gelten sowohl für sich genommen als auch insbesondere in Kombination miteinander.

Unter im Wesentlichen enantiomerenreinen Verbindungen der Formeln (I-S) und (I-R) soll im Rahmen der vorliegenden Erfindung verstanden werden, dass diese in einer Enantiomerenreinheit von jeweils wenigstens 95 % ee, vorzugsweise wenigstens 96 % ee und insbesondere wenigstens 97 % ee vorliegen.

Vorzugsweise wird in Schritt (i) des Verfahrens B ein Gemisch erhalten, in welchem das S-Enantiomer im Wesentlichen in nicht acylierter Form vorliegt und das R-Enantiomer in im Wesentlichen acylierter Form enthalten ist.

Unter "im Wesentlichen nicht acyliertes Enantiomer der Verbindung (II)" soll verstanden werden, dass wenigstens 95%, vorzugsweise wenigstens 97%, insbesondere wenigstens 98% dieses Enantiomers der Verbindung (II) nicht acyliert sind. Sinngemäß gilt für den Ausdruck "im Wesentlichen acyliertes Enantiomer der Verbindung (II)", dass wenigstens 95%, vorzugsweise wenigstens 97%, besonders bevorzugt wenigstens 98%, dieses Enantiomers der Verbindung (II) acyliert vorliegen.

Unter "im Wesentlichen nicht acyliertes S-Enantiomer der Verbindung (II)" soll dementsprechend verstanden werden, dass wenigstens 95%, vorzugsweise wenigstens 97%, insbesondere wenigstens 98% des S-Enantiomers der Verbindung (II) nicht acyliert sind. Sinngemäß gilt für den Ausdruck "im Wesentlichen acyliertes R-Enantiomer der Verbindung (II)", dass wenigstens 95%, vorzugsweise wenigstens 97%, besonders bevorzugt wenigstens 98%, des R-Enantiomers der Verbindung (II) acyliert vorliegen.

Bei der in Schritt (i) des Verfahrens B eingesetzten Hydrolase handelt es sich vorzugsweise um eine Protease und insbesondere um eine Lipase. Diese bewirkt eine selektive N-Acylierung (Amidierung) nur eines der beiden Enantiomere der Verbindung (II). Vorzugsweise bewirkt es die selektive Amidierung des R-Enantiomers der Verbindung (II). Die Hydrolase wird vorzugsweise aus einem Mikroorganismus, besonders bevorzugt aus einem Bakterium oder einer Hefe gewonnen. Ebenfalls geeignet sind Hydrolasen, die durch rekombinante Verfahren erhältlich sind. Die Hydrolase kann in gereinigter oder teilweise gereinigter Form oder in Form des Mikroorganismus selbst verwendet werden. Verfahren zur Gewinnung und Aufreinigung von Hydrolasen aus Mikroorganismen sind dem Fachmann hinreichend bekannt, z.B. aus EP-A-11149849 oder EP-A-1069183. Vorzugsweise wird die Hydrolase in gereinigter Form eingesetzt.

Die Hydrolase kann frei (d.h. in nativer Form) oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie).

Bevorzugt werden Lipasen (Triacylglycerolacylhydrolasen; EC 3.1.1.3) eingesetzt. Hierunter bevorzugt sind Lipasen, die aus Bakterien der Gattungen Burkholderia oder Pseudomonas oder aus Hefen der Gattung Candida gewonnen werden.

Beispiele für Burkholderia-Arten sind Burkholderia ambifaria (z. B. Stämme ATCC BAA-244, CCUG 44356, LMG 19182); Burkholderia andropogonis (z. B. Stämme ATCC 23061, CCUG 32772, CFBP 2421, CIP 105771, DSM 9511, ICMP 2807, JCM 10487, LMG 2129, NCPPB 934, NRRL B-14296); Burkholderia caledonica (z. B. Stämme W50D, CCUG 42236, CIP 107098, LMG 19076); Burkholderia caribensis (z. B. Stämme MWAP 64, CCUG 42847, CIP 106784, DSM 13236, LMG 18531); Burkholderia caryophylli (z.B. Stämme ATCC 25418, CCUG 20834, CFBP 2429, CFBP 3818, CIP 105770, DSM 50341, HAMBI 2159, ICMP 512, JCM 9310, JCM 10488, LMG 2155, NCPPB 2151); Burkholderia cepacia (z. B. Stämme Ballard 717, 717-ICPB 25, ATCC 25416, CCUG 12691, CCUG 13226, CFBP 2227, CIP 80.24, DSM 7288, HAMBI 1976, ICMP 5796, IFO 14074, JCM 5964, LMG 1222, NCCB 76047, NCPPB 2993, NCTC 10743, NRRL B-14810); Burkholderia cocovenenans (z. B. Stämme ATCC 33664, CFBP 4790, DSM 11318, JCM 10561, LMG 11626, NCIMB 9450); Burkholderia fungorum (z. B. Stämme Croize P763-2, CCUG 31961, CIP 107096, LMG 16225); Burkholderia gladioli (z. B. Stämme ATCC 10248, CCUG 1782, CFBP 2427, CIP 105410, DSM 4285, HAMBI 2157, ICMP 3950, IFO 13700, JCM 9311, LMG 2216, NCCB 38018, NCPPB 1891, NCTC 12378, NRRL B-793); Burkholderia glathei (z. B. Stämme ATCC 29195, CFBP 4791, CIP 105421, DSM 50014, JCM 10563, LMG 14190); Burkholderia glumae (z.B. Stämme ATCC 33617, CCUG 20835, CFBP 4900, CFBP 2430, CIP 106418, DSM 9512, ICMP 3655, LMG 2196, NCPPB 2981, NIAES 1169); Burkholderia graminis (z. B. Stämme C4D1M, ATCC 700544, CCUG 42231, CIP 106649, LMG 18924); Burkholderia kururiensis (z. B. Stämme KP 23, ATCC 700977, CIP 106643, DSM 13646, JCM 10599, LMG 19447); Burkholderia mallei (z. B. Stämme ATCC 23344, NCTC 12938); Burkholderia multivorans (z. B. Stämme ATCC BAA-247, CCUG 34080, CIP 105495, DSM 13243, LMG 13010, NCTC 13007); Burkholderia norimbergensis (z. B. Stämme R2, ATCC BAA-65, CCUG 39188, CFBP 4792, DSM 11628, CIP 105463, JCM 10565, LMG 18379); Burkholderia phenazinium (z. B. Stämme ATCC 33666, CCUG 20836, CFBP 4793, CIP 106502, DSM 10684, JCM 10564, LMG 2247, NCIB 11027); Burkholderia pikettii (z. B. Stämme ATCC 27511, CCUG 3318, CFBP 2459, CIP 73.23, DSM 6297, HAMBI 2158, JCM 5969, LMG 5942, NCTC 11149); Burkholderia plantarii (z. B. Stämme AZ 8201, ATCC 43733, CCUG 23368, CFBP 3573, CFBP 3997, CIP 105769, DSM 9509, ICMP 9424 JCM 5492, LMG 9035, NCPPB 3590, NIAES 1723); Burkholderia pseudomallei (z. B. Stämme WRAIR 286, ATCC 23343, NCTC 12939); Burkholderia pyrrocinia (z. B. Stämme ATCC 15958, CFBP 4794, CIP 105874, DSM 10685, LMG 14191); Burkholderia sacchari (z. B. Stämme CCT 6771, CIP 107211, IPT 101, LMG 19450); Burkholderia solanacearum (z. B. Stämme A. Kelman 60-1, ATCC 11696, CCUG 14272, CFBP 2047, CIP 104762, DSM 9544, ICMP 5712, JCM 10489, LMG 2299, NCAIM B.01459, NCPPB 325, NRRL B-3212); Burkholderia stabilis (z. B. Stämme ATCC BAA-67, CCUG 34168, CIP 106845, LMG 14294, NCTC 13011); Burkholderia thailandensis (z. B. Stämme E 264, ATCC 700388, CIP 106301, DSM 13276); Burkholderia ubonensis (z. B. Stämme EY 3383, CIP 107078, NCTC 13147); Burkholderia vandii (z. B. Stämme VA-1316, ATCC 51545, CFBP 4795, DSM 9510, JCM 7957, LMG 16020); Burkholderia vietnamiensis (z. B. Stämme TVV 75, ATCC BAA-248, CCUG 34169, CFBP 4796, CIP 105875, DSM 11319, JCM 10562, LMG 10929).

Beispiele für Pseudomonas-Arten sind Pseudomonas aeruginosa (z. B. Stämme ATCC 10145, DSM 50071), Pseudomonas agarici (z. B. Stämme ATCC 25941, DSM 11810), Pseudomonas alcaligenes (z. B. Stämme ATCC 14909, DSM 50342), Pseudomonas amygdali (z. B. Stämme ATCC 337614, DSM 7298), Pseudomonas anguiliseptica (z. B. Stämme ATCC 33660, DSM 12111), Pseudomonas antimicrobica (z. B. Stämme DSM 8361, NCIB 9898, LMG 18920), Pseudomonas aspleni (z. B. Stämme ATCC 23835, CCUG 32773), Pseudomonas aurantiaca (z. B. Stämme ATCC 33663, CIP 106710), Pseudomonas aureofaciens (z. B. Stämme ATCC 13985, CFBP 2133), Pseudomonas avellanae (z. B. Stämme DSM 11809, NCPPB 3487), Pseudomonas azotoformans (z. B. Stämme CIP 106744, JCM 7733), Pseudomonas balearica (z. B. Stämme DSM 6083, CIP 105297)), Pseudomonas beijerinsckii (z. B. Stämme ATCC 19372, DSM 6083), Pseudomonas beteli (z. B. Stämme ATCC 19861, CFBP 4337), Pseudomonas boreopolis (z. B. Stämme ATCC 33662, CIP 106717), Pseudomonas carboxyhydrogena (z. B. Stämme ATCC 29978, DSM 1083), Pseudomonas caricapapayae (z. B. Stämme ATCC 33615, CCUG 32775), Pseudomonas cichorii (z. B. Stämme ATCC 10857, DSM 50259), Pseudomonas cissicola (z. B. Stämme ATCC 33616, CCUG 18839), Pseudomonas citronellolis (z. B. Stämme ATCC 13674, DSM 50332), Pseudomonas coronafaciens (z. B. Stämme DSM 50261, DSM 50262), Pseudomonas corrugata (z. B. Stämme ATCC 29736, DSM 7228), Pseudomonas doudoroffii (z. B. Stämme ATCC 27123, DSM 7028), Pseudomonas echinoides (z. B. Stämme ATCC 14820, DSM 1805), Pseudomonas elongata (z. B. Stämme ATCC 10144, DSM 6810), Pseudomonas ficuserectae (z. B. Stämme ATCC 35104, CCUG 32779), Pseudomonas flavescens (z. B. Stämme ATCC 51555, DSM 12071), Pseudomonas flectens (z. B. Stämme ATCC 12775, CFBB 3281), Pseudomonas fluorescens (z. B. Stämme ATCC 13525, DSM 50090), Pseudomonas fragi (z. B. Stämme ATCC 4973, DSM 3456), Pseudomonas fulva (z. B. Stämme ATCC 31418, CIP 106765), Pseudomonas fuscovaginae (z. B. Stämme CCUG 32780, DSM 7231), Pseudomonas gelidicola (z. B. Stämme CIP 106748), Pseudomonas geniculata (z. B. Stämme ATCC 19374, LMG 2195), Pseudomonas glathei (z. B. Stämme ATCC 29195, DSM 50014), Pseudomonas halophila (z. B. Stämme ATCC 49241, DSM 3050), Pseudomonas hibiscicola (z. B. Stämme ATCC 19867, LMG 980), Pseudomonas huttiensis (z. B. Stämme ATCC 14670, DSM 10281), Pseudomonas iners (z. B. Stamm CIP 106746), Pseudomonas lancelota (z. B. Stämme ATCC 14669, CFBP 5587), Pseudomonas lemoignei (z. B. Stämme ATCC 17989, DSM 7445), Pseudomonas lundensis (z. B. Stämme ATCC 19968, DSM 6252), Pseudomonas luteola (z. B. Stämme ATCC 43273, DSM 6975), Pseudomonas marginalis (z. B. Stämme ATCC 10844, DSM 13124), Pseudomonas meliae (z. B. Stämme ATCC 33050, DSM 6759), Pseudomonas mendocina (z. B. Stämme ATCC 25411, DSM 50017), Pseudomonas mucidolens (z. B. Stämme ATCC 4685, CCUG 1424), Pseudomonas monteilli (z. B. Stämme ATCC 700476, DSM 14164), Pseudomonas nautica (z. B. Stämme ATCC 27132, DSM 50418), Pseudomonas nitroreducens (z. B. Stämme ATCC 33634, DSM 14399), Pseudomonas oleovorans (z. B. Stämme ATCC 8062, DSM 1045), Pseudomonas oryzihabitans (z. B. Stämme ATCC 43272, DSM 6835), Pseudomonas pertucinogena (z. B. Stämme ATCC 190, CCUG 7832), Pseudomonas phenazinium (z. B. Stämme ATCC 33666, DSM 10684), Pseudomonas pictorum (z. B. Stämme ATCC 23328, LMG 981), Pseudomonas pseudoalcaligenes (z. B. Stämme ATCC 17440, DSM 50188), Pseudomonas putida (z. B. Stämme ATCC 12633, DSM 291), Pseudomonas pyrrocinia (z. B. Stämme ATCC 15958, DSM 10685), Pseudomonas resinovorans (z. B. Stämme ATCC 14235, CCUG 2473), Pseudomonas rhodesiae (z. B. Stämme CCUG 38732, DSM 14020), Pseudomonas saccharophila (z. B. Stämme ATCC 15946, DSM 654), Pseudomonas savastanoi (z. B. Stämme ATCC 13522, CFBP 1670), Pseudomonas spinosa (z. B. Stämme ATCC 14606), Pseudomonas stanieri (z. B. Stämme ATCC 27130, DSM 7027), Pseudomonas straminae (z. B. Stämme ATCC 33636, CIP 106745), Pseudomonas stutzeri (z. B. Stämme ATCC 17588, DSM 5190), Pseudomonas synxantha (z. B. Stämme ATCC 9890, CFBP 5591), Pseudomonas syringae (z. B. Stämme ATCC 19310, DSM 6693), Pseudomonas syzygii (z. B. Stämme ATCC 49543, DSM 7385), Pseudomonas taetrolens (z. B. Stämme ATCC 4683, CFBP 5592), Pseudomonas tolaasii (z. B. Stämme ATCC 33618, CCUG 32782), Pseudomonas veronii (z. B. Stämme ATCC 700272, DSM 11331), Pseudomonas viridiflava (z. B. Stämme ATCC 13223, DSM 11124), Pseudomonas vulgaris, Pseudomonas wisconsinensis und Pseudomonas spec. DSM 8246. Davon sind Lipasen aus Burkholderia glumae, Burkholderia plantarii, Burkholderia cepacia, Pseudomonas aeruginosa, Pseudomonas fluorescens, Pseudomonas fragi, Pseudomonas luteola, Pseudomonas vulgaris, Pseudomonas wisconsinensis und Pseudomonas spec. DSM 8246 bevorzugt. Besonders bevorzugt sind Lipasen aus Pseudomonas spec. DSM 8246.

Beispiele für Candida-Arten sind Candida albomarginata (z.B. Stamm DSM 70015), Candida antarctica (z.B. Stamm DSM 70725), Candida bacarum (z.B. Stamm DSM 70854), Candida bogoriensis (z.B. Stamm DSM 70872), Candida boidinii (z.B. Stämme DSM 70026, 70024, 70033, 70034 ), Candida bovina (z.B. Stamm DSM 70156), Candida brumptii (z.B. Stamm DSM 70040), Candida cacaoi (z.B. Stamm DSM 2226), Candida cariosilignicola (z.B. Stamm DSM 2148), Candida chalmersii (z.B. Stamm DSM 70126), Candida ciferii (z.B. Stamm DSM 70749), Candida cylindracea (z.B. Stamm DSM 2031), Candida ernobii (z.B. Stamm DSM 70858), Candida famata (z.B. Stamm DSM 70590), Candida freyschussii (z.B. Stamm DSM 70047), Candida friederichii (z.B. Stamm DSM 70050), Candida glabrata (z.B. Stämme DSM 6425, 11226, 70614, 70615), Candida guillermondi (z.B. Stämme DSM 11947, 70051, 70052), Candida haemulonii (z.B. Stamm DSM 70624), Candida inconspicua (z.B. Stamm DSM 70631), Candida ingens (z.B. Stämme DSM 70068, 70069), Candida intermedia (z.B. Stamm DSM 70753), Candida kefyr (z.B. Stämme DSM 70073, 70106), Candida krusei (z.B. Stämme DSM 6128, 11956, 70075, 70079, 70086), Candida lactiscondensi (z.B. Stamm DSM 70635), Candida lambica (z.B. Stämme DSM 70090, 70095), Candida lipolytica (z.B. Stämme DSM 1345, 3286, 8218, 70561 oder 70562), Candida lusitaniae (z.B. Stamm DSM 70102), Candida macedoniensis (z.B. Stamm DSM 70106), Candida magnoliae (z.B. Stämme DSM 70638, 70639), Candida membranaefaciens (z.B. Stamm DSM 70109), Candida multigemnis (z.B. Stamm DSM 70862), Candida mycoderma (z.B. Stamm DSM 70184), Candida nemodendra (z.B. Stamm DSM 70647), Candida nitratophila (z.B. Stamm DSM 70649), Candida norvegica (z.B. Stamm DSM 70862), Candida parapsilosis (z.B. Stämme DSM 5784, 4237, 11224, 70125, 70126), Candida pelliculosa (z.B. Stamm DSM 70130), Candida pini (z.B. Stamm DSM 70653), Candida pulcherrima (z.B. Stamm DSM 70336), Candida punicea (z.B. Stamm DSM 4657), Candida pustula (z.B. Stamm DSM 70865), Candida rugosa (z.B. Stamm DSM 70761), Candida sake (z.B. Stamm DSM 70763), Candida silvicola (z.B. Stamm DSM 70764), Candida solani (z.B. Stamm DSM 3315), Candida sp. (z.B. Stamm DSM 1247), Candida spandovensis (z.B. Stamm DSM 70866), Candida succiphila (z.B. Stamm DSM 2149), Candida utilis (z.B. Stämme DSM 2361, 70163 oder 70167), Candida valida (z.B. Stämme DSM 70169, 70178, 70179), Candida versatilis (z.B. Stamm DSM 6956), Candida vini (z.B. Stamm DSM 70184) und Candida zeylanoides (z.B. Stamm DSM 70185).

Besonders bevorzugt werden im erfindungsgemäßen Verfahren Lipasen aus Hefen der Gattung Candida eingesetzt, insbesondere aus Candida antarctica. In einer speziellen Ausführungsform wird Lipase B aus Candida antarctica verwendet. Bevorzugt ist die immobilisierte Form dieser Lipase, z.B. die auf Acrylharz immobilisierte Lipase B aus Candida antarctica, die z.B. unter der Bezeichnung "Novozym 435^{®}" kommerziell erhältlich ist.

Die in Schritt (i) des Verfahrens B eingesetzten Acylierungsmittel sind vorzugsweise unter solchen ausgewählt, in denen die Säurekomponente ein elektronenreiches Heteroatom, das beispielsweise ausgewählt ist unter Fluor-, Stickstoff-, Sauerstoff- und Schwefelatomen, in Nachbarschaft zum Carbonylkohlenstoffatom trägt. Vorzugsweise handelt es sich bei dem Acylierungsmittel um einen Ester. Besonders bevorzugt ist als Acylierungsmittel ausgewählt unter Estern, deren Säurekomponente in α-, β- oder γ-Stellung zum Carbonyl-Kohlenstoffatom ein eine sauerstoff-, stickstoff-, fluor- oder schwefelhaltige Gruppe trägt. Besonders bevorzugt sind solche Acylierungsmittel, in denen das Heteroatom selbst in α-, β- oder γ-Stellung und insbesondere in α-Stellung zum Carbonylkohlenstoffatom gebunden ist.

Bei der sauerstoffhaltigen Gruppe handelt es sich beispielsweise um eine Hydroxygruppe oder um eine Alkoxygruppe. Bei der stickstoffhaltigen Gruppe handelte es sich beispielsweise um Aminogruppen, während es sich bei der schwefelhaltigen Gruppe um die Thiolgruppe (SH) oder um Thioalkylgruppen handeln kann.

Die Alkoholkomponente des Esters leitet sich vorzugsweise von linearen oder verzweigten C₁-C₁₀-Alkoholen, die substituiert oder vorzugsweise unsubstituiert sein können, ab. Besonders bevorzugt leitet sich die Alkoholkomponente jedoch von sekundären Alkoholen ab, wie Isopropanol, 2-Butanol, 2- oder 3-Pentanol und dergleichen. Speziell leitet sie sich von Isopropanol ab.

Besonders geeignete Ester sind solche der Formel V 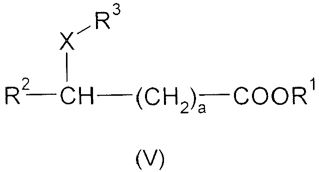 worin
- R¹: für C₁-C₁₀-Alkyl steht,
- R²: für Wasserstoff oder C₁-C₁₀-Alkyl steht,
- R³: für Wasserstoff, C₁-C₁₀-Alkyl oder Phenyl, das gegebenenfalls durch NH₂, OH, C₁-C₄-Alkoxy oder Halogen substituiert ist, steht,
- X: für O, S oder NR⁴ steht,
- R⁴: für Wasserstoff, C₁-C₁₀-Alkyl oder Phenyl, das gegebenenfalls durch NH₂, OH, C₁-C₄-Alkoxy oder Halogen substituiert ist, steht, und
- a: für 0, 1 oder 2 steht.

R¹ steht vorzugsweise für lineares oder verzweigtes C₁-C₄-Alkyl. Besonders bevorzugt leitet sich R¹ von sekundären Alkoholen ab und steht dementsprechend besonders bevorzugt für eine über ein tertiäres Kohlenstoffatom gebundene C₃-C₄-Alkylgruppe, wie Isopropyl oder 2-Butyl. Insbesondere steht R¹ für Isopropyl.

X steht vorzugsweise für O.

R² steht vorzugsweise für Wasserstoff oder C₁-C₄-Alkyl und insbesondere für Wasserstoff.

R³ steht vorzugsweise für C₁-C₄-Alkyl, besonders bevorzugt für Methyl oder Ethyl und speziell für Methyl.

Im Rahmen der vorliegenden Erfindung steht C₁-C₄-Alkyl für einen linearen oder verzweigten Alkylrest mit 1 bis 4 Kohlenstoffatomen, wie Methyl, Ethyl, Propyl, Isopropyl, n-Butyl, sec-Butyl, Isobutyl oder tert-Butyl.

C₁-C₆-Alkyl steht für einen linearen oder verzweigten Alkylrest mit 1 bis 6 Kohlenstoffatomen. Beispiele hierfür sind neben den zuvor genannten C₁-C₄-Alkylresten Pentyl, Neopentyl und Hexyl.

C₁-C₁₀-Alkyl steht für einen linearen oder verzweigten Alkylrest mit 1 bis 10 Kohlenstoffatomen. Beispiele hierfür sind neben den zuvor genannten C₁-C₆-Alkylresten Heptyl, Octyl, 2-Ethylhexyl, Nonyl, Neononyl, Decyl und Neodecyl.

C₁-C₄-Alkoxy steht für einen über Sauerstoff gebundenen Alkylrest mit 1 bis 4 Kohlenstoffatomen. Beispiele hierfür sind Methoxy, Ethoxy, Propoxy, Isopropoxy, n-Butoxy, sec-Butoxy, Isobutoxy und tert-Butoxy.

C₁-C₁₀-Alkohol steht für einen aliphatischen Kohlenwasserstoff mit 1 bis 10 Kohlenstoffatomen, der durch wenigstens eine Hydroxygruppe substituiert ist. Vorzugsweise steht C₁-C₁₀-Alkohol für ein mit einem Hydroxyrest substituiertes Alkan. Beispiele hierfür sind Methanol, Ethanol, Propanol, Isopropanol, Butanol, sec-Butanol, Isobutanol, tert-Butanol, Pentanol, Hexanol, Heptanol, Octanol, 2-Ethylhexanol, Nonanol und Decanol.

Halogen steht im Rahmen der vorliegenden Erfindung für vorzugsweise Fluor, Chlor oder Brom, besonders bevorzugt für Fluor oder Chlor.

In Verbindungen der Formel (I-S) und (I-R) sowie (II) steht n vorzugsweise für 0 oder 2 und insbesondere für 0.

m steht vorzugsweise für 0 oder 1 und insbesondere für 0.

Die Hydroxyfunktion in Verbindungen (I-S) und (I-R) bzw. die geschützte Hydroxyfunktion (PO-) in Verbindungen (II) kann in ortho, meta- oder para-Position zur Aminoalkylgruppe (-(CH₂)ₙ-CH(NH₂)-(CH₂)ₘ-CH₃) stehen. Vorzugsweise steht die Hydroxyfunktion in Verbindungen (I-S) und (I-R) bzw. die geschützte Hydroxyfunktion (PO-) in Verbindungen (II) in meta- oder para-Position und insbesondere in para-Position zur Aminoalkylgruppe.

In Verbindungen (II) steht die Schutzgruppe P bevorzugt für tert-Butyl oder für einen Benzylrest und insbesondere für tert-Butyl.

Vorzugsweise setzt man bei der Umsetzung in Schritt (i) des Verfahrens B 1 bis 5 Moläquivalente, besonders bevorzugt 1 bis 4 Moläquivalente und insbesondere 1 bis 3 Moläquivalente des Acylierungsmittels, bezogen auf den Gehalt an Enantiomer der Verbindung der Formel (II), das acyliert wird, ein. Unter Moläquivalenten soll die Anzahl der Carboxylgruppen des Acylierungsmittels in Mol verstanden werden, die mit 1 mol desjenigen Enantiomers der Verbindung (II), das acyliert wird, reagieren können. Dementsprechend setzt man bei der Verwendung der Ester der Formel (V) vorzugsweise 1 bis 5 mol, besonders bevorzugt 1 bis 4 mol und insbesondere 1 bis 3 mol Ester, bezogen auf 1 mol Enantiomer der Verbindung der Formel (II), das acyliert wird, ein. Alternativ setzt man bei der Verwendung der Ester der Formel (V) vorzugsweise 0,5 bis 2,5 mol, besonders bevorzugt 0,5 bis 2 mol und insbesondere 0,5 bis 1,5 mol Ester, bezogen auf 1 mol Enantiomerengemisch der Formel (II), ein.

Die zuzusetzende Menge an Hydrolase hängt von deren Art und der Aktivität der Enzympräparation ab. Die für die Reaktion optimale Enzymmenge kann leicht durch einfache Vorversuche ermittelt werden. In der Regel werden 1000 units Hydrolase/mmol der Verbindung (II) eingesetzt.

In einer bevorzugten Ausführungsform wird die Umsetzung in Schritt (i) des Verfahrens B in einem nicht-wässrigen Reaktionsmedium durchgeführt. Unter nicht-wässrigen Reaktionsmedien sollen Reaktionsmedien verstanden werden, die weniger als 1 Gew.-%, vorzugsweise weniger als 0,5 Gew.-% Wasser, besonders bevorzugt weniger als 0,1 Gew.-% Wasser und insbesondere weniger als 0,05 Gew.-% Wasser, bezogen auf das Gesamtgewicht des Reaktionsmediums, enthalten. Vorzugsweise wird die Umsetzung in einem organischen Lösungsmittel durchgeführt. Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit 1 oder 2 Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Dioxan, oder Gemische der vorgenannten Lösungsmittel. Besonders bevorzugt werden die vorgenannten Ether und aromatischen Kohlenwasserstoffe eingesetzt. Insbesondere verwendet man Toluol.

In einer alternativ bevorzugten Ausführungsform erfolgt die Umsetzung in Verfahren A bzw. in Schritt (i) des Verfahrens B in Substanz, d.h. ohne wässriges oder organisches Lösungsmittel.

Die Umsetzung in Schritt (i) des Verfahrens B erfolgt in der Regel bei einer Reaktionstemperatur unterhalb der Deaktivierungstemperatur der eingesetzten Hydrolase und vorzugsweise bei wenigstens -10°C. Besonders bevorzugt liegt sie im Bereich von 0 bis 80°C, insbesondere von 20 bis 40°C. Speziell erfolgt die Umsetzung bei Raumtemperatur.

Zur Durchführung kann man beispielsweise das Enantiomerengemisch der Formel (II) mit der Hydrolase, dem Acylierungsmittel und gegebenenfalls dem Lösungsmittel vorlegen und das Gemisch durchmischen, z.B. durch Rühren oder Schütteln. Es ist aber auch möglich, die Hydrolase in einem Reaktor, beispielsweise in einer Säule, zu immobilisieren, und durch den Reaktor eine das Enantiomerengemisch und das Acylierungsmittel enthaltende Mischung zu leiten. Hierzu kann man die Mischung im Kreislauf durch den Reaktor leiten, bis der gewünschte Umsatz erreicht ist. Dabei werden die Carboxylgruppen des Acylierungsmittels sequenziell in Amide desjenigen Enantiomers der Verbindung (II), welches enantioselektiv acyliert wird, überführt, während das andere Enantiomer im Wesentlichen unverändert bleibt. In der Regel wird man die Acylierung bis zu einem Umsatz von wenigstens 95%, bevorzugt von wenigstens 99% und insbesondere von wenigstens 99,5%, bezogen auf das in der Mischung enthaltene Enantiomer der Verbindung (II), welches enantioselektiv acyliert wird, führen. Das Fortschreiten der Reaktion, d.h. die sequenzielle Amidbildung, kann dabei durch übliche Methoden wie Gaschromatographie oder HPLC (High Performance Liquid Chromatography) verfolgt werden.

Beim eingesetzten Enantiomerengemisch (II) handelt es sich in der Regel um das Racemat der Amine; Gemische, in denen eines der Enantiomere angereichert ist, sind jedoch auch geeignet.

Die Aufarbeitung des Reaktionsgemischs kann in üblicher Weise erfolgen, beispielsweise, indem man zunächst die Hydrolase aus dem Reaktionsgemisch abtrennt, z.B. durch Abfiltrieren oder Abzentrifugieren, gegebenenfalls aus dem Filtrat bzw. Zentrifugat das Lösungsmittel entfernt und den Rückstand anschließend einer Trennoperation unterwirft.

Durch die enantioselektive Umsetzung des Enantiomerengemisches der Formel (II) entsteht ein Reaktionsprodukt, das im Wesentlichen ein acyliertes Enantiomer (d.h. Amid) der Verbindung (II) und das im Wesentlichen nicht acylierte entgegengesetzte Enantiomer enthält. Dieses nun vorliegende Gemisch aus Amin und Amid lässt sich mit üblichen Methoden leicht trennen. Geeignete Trennoperationen sind beispielsweise Extraktion, Destillation, Kristallisation oder Chromatographie. Vorzugsweise erfolgt die Trennung des Amins und des Amids destillativ. In einem alternativ bevorzugten Trennverfahren wird das in einem organischen Lösungsmittel gelöste oder suspendierte Reaktionsgemisch mit einer Säure versetzt, wobei sich das Ammoniumsalz des nicht acylierten Enantiomers bildet und präzipitiert. Dieses kann dann durch Filtration oder Zentrifugieren vom Überstand, welcher das Amid des einen Enantiomers der Verbindung (II) enthält, abgetrennt werden. Geeignete Säuren sind beispielsweise Mineralsäuren, wie Salzsäure, Bromwasserstoffsäure, Schwefelsäure oder Salpetersäure. Geeignet sind aber auch organische Säuren, wie Trifluoressigsäure oder Trifluormethansulfonsäure, wobei jedoch Mineralsäuren und insbesondere Schwefelsäure bevorzugt sind. Die Säure wird vorzugsweise in einer solchen Menge eingesetzt, dass solche Schutzgruppen P der OH-Funktion, die üblicherweise durch Säurebehandlung abgespalten werden, nicht entfernt werden. Dies gilt insbesondere bei der Verwendung von tert-Butyl als Schutzgruppe. Vorzugsweise wird die Säure in etwa äquimolarer Menge zum nicht acylierten Enantiomer eingesetzt, z.B. in einer Menge von 0,9 bis 1,5 mol, vorzugsweise 1 bis 1,2 mol und insbesondere etwa 1 mol, bezogen auf 1 mol des nicht acylierten Enantiomers. Bei mehrprotonigen Säuren, wie Schwefelsäure, beziehen sich die Molverhältnisse selbstverständlich auf die Anzahl der in der Säure enthaltenen Protonen.

In Schritt (iii) wird schließlich das in Schritt (ii) abgetrennte, im Wesentlichen nicht acylierte Enantiomer der Verbindung (II) entschützt und in das entsprechende freie im Wesentlichen enantiomerenreine Amin überführt. Insbesondere handelt es sich bei dem in Schritt (ii) abgetrennten nicht acylierten Enantiomer um das S-Enantiomer der Verbindung (II), das in Schritt (iii) entschützt und in die Verbindung (I-S) überführt wird.

Das Entschützen erfolgt in der Regel unter solchen Reaktionsbedingungen, wie sie aus dem Stand der Technik für die Abspaltung der jeweiligen Schutzgruppe P bekannt sind. Geeignete Entschützungsverfahren sind beispielsweise in T.W. Greene, P.G.M. Wuts, Protective Groups in Organic Synthesis, 3. Auflage, Wiley Interscience 1999 oder in P.J. Kocienski, Protective Groups, Thieme 2000 beschrieben, worauf hiermit in vollem Umfang Bezug genommen wird.

So erfolgt die Abspaltung der tert-Butyl-Schutzgruppe vorzugsweise durch Behandlung der geschützten Verbindung mit einer Säure. Geeignete Säuren sind die oben genannten mineralischen und organischen Säuren. Bevorzugte Säuren sind mineralische Säuren, wie Salzsäure und Schwefelsäure. Vorzugsweise erfolgt die Entschützungsoperation mit Säuren bei erhöhten Reaktionstemperaturen, z.B. bei 50 bis 150°C, vorzugsweise 70 bis 100°C.

Wurde eine benzylische Schutzgruppe P eingesetzt, so erfolgt die Abspaltung der Benyzlkomponente vorzugsweise hydrogenolytisch. Die Hydrogenolyse erfolgt in der Regel unter bekannten Bedingungen, beispielsweise unter Verwendung eines geeigneten Hydrierkatalysators, wie Palladium, Palladiumhydroxid oder Platin.

Liegt das Amin nach der Schutzgruppenabspaltung noch in Form des Ammoniumsalzes vor, beispielsweise weil das geschützte Produkt mit Säuren entschützt wurde oder weil die Abtrennung in Schritt (ii) durch Präzipitation des Ammoniumsalzes erfolgt ist, so wird das Amin aus dem Ammoniumsalz mittels einer geeigneten Base freigesetzt. Geeignete Basen sind beispielsweise Alkalihydroxide, wie Natriumhydroxid oder Kaliumhydroxid, Erdalkalihydroxide, wie Calciumhydroxid oder Magnesiumhydroxid, Alkalicarbonate oder Erdalkalicarbonate, wie Natriumcarbonat, Kaliumcarbonat oder Calciumcarbonat. Bevorzugt verwendet man Alkalihydroxide, wie Natriumhydroxid oder Kaliumhydroxid. Vorzugsweise erfolgt die Neutralisation in einem wässrigen Medium. Zur leichteren Isolierung des Amins wird die Base vorzugsweise in einer solchen Menge eingesetzt, dass einerseits die Aminfunktion neutralisiert wird, andererseits aber die Hydroxyfunktion am Phenolring nicht deprotoniert wird und das neutrale Molekül präzipitiert. Dementsprechend wird der pH-Wert bevorzugt auf den isoelektrischen Punkt eingestellt. Das gewonnene freie Amin kann anschließend gewünschtenfalls weiteren Reinigungsschritten unterworfen werden.

Das andere Enantiomer, dessen geschütztes Derivat in Verfahren A bzw. in Schritt (i) des Verfahrens B enantioselektiv acyliert wurde, kann dadurch gewonnen werden, dass man das in Schritt (ii) gewonnene im Wesentlichen acylierte Enantiomer der Verbindung (II) unter Abspaltung der Acylfunktion hydrolysiert, wobei das entsprechende Enantiomer der Verbindung (II) erhalten wird. Vorzugsweise handelt es sich dabei um das R-Enantiomer der Verbindung (II).

Die Hydrolyse erfolgt dabei in der Regel unter Reaktionsbedingungen, wie sie für die Hydrolyse von Amiden bekannt sind. Reaktionsbedingungen sind beispielsweise in DE-A-19534208 oder in Organikum, VEB Deutscher Verlag der Wissenschaften, Berlin 1988, 17. Aufl., S. 419 oder in Jerry March, Advanced Organic Chemistry, 3. Aufl., John Wiley and Sons, S. 338 ff. beschrieben, worauf hiermit in vollem Umfang Bezug genommen wird. Vorzugsweise erfolgt die Hydrolyse zum Amin durch Umsetzung mit einer Base. Geeignete Basen sind beispielsweise Alkalihydroxide, wie Natriumhydroxid und Kaliumhydroxid, Erdalkalihydroxide, wie Calciumhydroxid, Alkali- und Erdalkalicarbonate, wie Natrium-, Kalium und Calciumcarbonat, Ammoniak, Amine, wie Dimethylamin, Diethylamin, Trimethylamin, Diethylamin, Triethylamin, Diisopropylamin und Diisopropylethylamin, oder Aminoalkohole, wie Ethanolamin, Diethanolamin und Triethanolamin. Besonders bevorzugt verwendet man die genannten Alkalihydroxide, gegebenenfalls in Kombination mit einem Amin oder Aminoalkohol. Die Hydrolyse kann in Wasser oder in einem Lösungsmittel oder in einer Mischung aus Wasser und Lösungsmittel durchgeführt werden. Geeignete Lösungsmittel sind Alkohole, vorzugsweise mit 1 bis 3 Kohlenstoffatomen, wie Methanol, Ethanol, Propanol oder Isopropanol, Glykole, insbesondere mit 2 bis 8 Kohlenstoffatomen, wie Ethylenglykol, Di- und Triethylenglykol, Amine und Aminoalkohole, z.B. die zuvor genannten Amine und Aminoalkohole, außerdem die Gemische der vorstehend genannten Lösungsmittel sowie deren Gemische mit Wasser. Die Hydrolyse erfolgt vorzugsweise bei erhöhter Temperatur, z.B. bei der Siedetemperatur des verwendeten Lösungsmittels. Das erhaltene hydrolysierte Enantiomer der Verbindung (II) kann anschließend unter den gleichen Reaktionsbedingungen, wie sie für das entgegengesetzte Enantiomer beschrieben sind, entschützt werden und in das entsprechende Amin (I-S) oder vorzugsweise (I-R) überführt werden.

Mit dem erfindungsgemäßen Verfahren werden die Amine (I-S) und (I-R) mit einem Enantiomerenüberschuss (ee-Wert) von vorzugsweise wenigstens 95%, besonders bevorzugt von wenigstens 96%, stärker bevorzugt von wenigstens 97% und insbesondere von wenigstens 98%, z.B. wenigstens 99% erhalten.

Speziell im Fall der Verbindung 4-(1-Aminoethyl)-phenol wird mit dem erfindungsgemäßen Verfahren das S-Enantiomer mit einem Enantiomerenüberschuss (ee-Wert) von vorzugsweise wenigstens 98% ee, besonders bevorzugt von wenigstens 99% ee und insbesondere von wenigstens 99,4% ee erhalten. Die Enantiomerenreinheit des entsprechenden R-Enantiomers beträgt vorzugsweise wenigstens 98% ee und besonders bevorzugt wenigstens 99% ee.

Speziell im Fall der Verbindung 3-(1-Aminoethyl)-phenol wird mit dem erfindungsgemäßen Verfahren das S-Enantiomer mit einem Enantiomerenüberschuss (ee-Wert) von vorzugsweise wenigstens 98% ee, besonders bevorzugt von wenigstens 99% ee und insbesondere von wenigstens 99,5% ee erhalten. Die Enantiomerenreinheit des entsprechenden R-Enantiomers beträgt vorzugsweise wenigstens 98% ee und besonders bevorzugt wenigstens 98,5% ee.

Der Enantiomerenüberschuss der Amine (I-S) und (I-R) kann mittels gängiger Verfahren bestimmt werden, beispielsweise durch Bestimmung der optischen Drehung oder durch Chromatographie an einer chiralen Phase, beispielsweise durch HPLC oder Gaschromatographie über chirale Säulen.

Wenn an einem der Enantiomere (I-R) oder (I-S) oder an Umsetzungsprodukten davon kein Interesse besteht, so kann dieses racemisiert und erneut oder in Schritt (i) des

Verfahrens B eingesetzt werden. Durch diese Rückführung wird es möglich, insgesamt mehr als 50% des gewünschten Enantiomers (I-S) oder (I-R) aus dem Enantiomerengemisch (II) zu erhalten. Geeignete Racemisierungsbedinungen sind bekannt und beispielsweise in der WO 00/209357 oder WO 00/47546 beschrieben, auf deren Inhalt hiermit ausdrücklich Bezug genommen wird.

Die Verbindung der Formel (II) wird dadurch erhalten, dass man
(a) eine Verbindung der Formel (III) 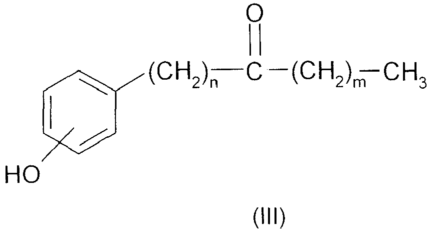 worin n und m wie vorstehend definiert sind,
   unter Erhalt der Verbindung (IV) 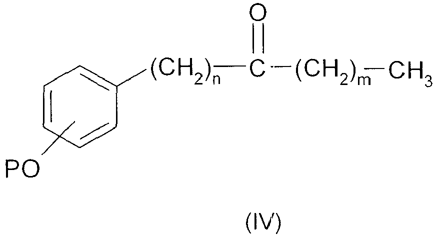 worin P wie vorstehend definiert ist,
   an der OH-Gruppe schützt,
   und
(b) die Verbindung (IV) einer reduktiven Aminierung unterwirft, wobei man die Verbindung der Formel (II) erhält.

Denkbar ist auch die umgekehrte Reihenfolge dieser Schritte, d.h. zuerst reduktive Aminierung der Verbindung (III) unter Erhalt einer Aminverbindung VI 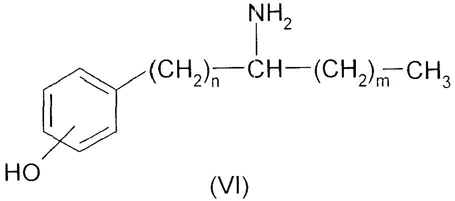 und erst anschließend Einführung der Hydroxy-Schutzgruppe am erhaltenen Amin VI unter Erhalt der Verbindung (II).

Geeignete Schutzgruppen P sind solche, die sich einerseits leicht anbringen lassen, andererseits aber auch die Schritte (i) und (ii) des Verfahrens B überstehen und sich außerdem, z.B. in den Schritten (iii) und (iv) des Verfahrens B, dennoch gut wieder entfernen lassen. Übliche Schutzgruppen für Hydroxyfunktionen sind die entsprechenden Alkylether, Benzylether und Silylether. Sie werden meist durch Umsetzung des entsprechenden Alkyl-, Benzyl- oder Silylhalogenids mit der zu schützenden Verbindung erhalten. Im Fall der Verbindung (III) hat es sich jedoch als besonders günstig herausgestellt, diese in den entsprechenden Benzylether oder einen am Phenylring der Benzylgruppe substituierten Benzylether oder insbesondere in den tert-Butylether zu überführen (P = Benzyl, substituiertes Benzyl oder tert-Butyl).

Bei der substituierten Benzylgruppe handelt es sich vorzugsweise um Methyl- oder Dimethylbenzylgruppen. Der Vorteil der Verwendung derartiger substituierter Benzylgruppen liegt in ihrer geringeren Empfindlichkeit gegenüber hydrogenolytischen Bedingungen des Schritts (b) im Vergleich zum unsubstituierten Benzyl.

Die Einführung der Schutzgruppe in Schritt (a) erfolgt in der Regel unter den üblichen Reaktionsbedingungen wie sie aus dem Stand der Technik für die Einführung der entsprechenden Schutzgruppe bekannt sind. In diesem Zusammenhang wird auf T.W. Greene, P.G.M. Wuts, Protective Groups in Organic Synthesis, 3. Auflage, Wiley Interscience 1999 sowie auf P.J. Kocienski, Protective Groups, Thieme 2000 verwiesen, worauf hiermit in vollem Umfang Bezug genommen wird

So erfolgt die Überführung der Verbindung (III) in den entsprechenden Benzylether beispielsweise durch Umsetzung von (III) mit einem gegebenenfalls am Phenylring substituierten Benzylhalogenid, z.B. mit Benzylbromid oder Benzylchlorid, wobei die Umsetzung in der Regel in Gegenwart einer Base durchgeführt wird. Geeignete Basen sind die zuvor genannten Alkali- und Erdalkalihydroxide sowie Alkali- und Erdalkalicarbonate.

Die Überführung in den entsprechenden tert-Butylether erfolgt vorzugsweise durch Umsetzen der Verbindung (III) mit Isobuten in Gegenwart einer Säure. Geeignete Säuren sind Mineralsäuren, wie Schwefelsäure, und starke organische Säuren, wie Trifluoressigsäure oder Trifluormethansulfonsäure. Besonders bevorzugt verwendet man Trifluormethansulfonsäure. Die Säure wird dabei in katalytischen Mengen eingesetzt. Isobuten wird in wenigstens äquimolarer Menge in Bezug auf die schützende Verbindung eingesetzt, z.B. in einer Menge von 1 bis 10 Mol, bezogen auf 1 Mol der zu schützenden Verbindung. Vorzugsweise wird es jedoch im Überschuss eingesetzt. Vorzugsweise beträgt das Molverhältnis von Isobuten zur zu schützenden Verbindung 2:1 bis 10:1, besonders bevorzugt 3:1 bis 7:1, z.B. etwa 5:1. Die Schutzgruppenoperation erfolgt in der Regel in einem Lösungsmittel. Geeignete Lösungsmittel sind solche, die weder mit den Reaktanden noch mit dem Produkt eine Reaktion eingehen. Beispiele hierfür sind die zu Schritt (i) genannten Lösungsmittel. Bevorzugt verwendet man halogenierte Alkane, wie Methylenchlorid, Chloroform, Tetrachlorkohlenstoff oder Dichlorethan. Die Reaktionstemperatur beträgt üblicherweise -80 bis 40°C, besonders bevorzugt-50 bis 30°C und insbesondere etwa Raumtemperatur. Die Umsetzung kann sowohl drucklos als auch unter erhöhtem Druck erfolgen. Bei einer drucklosen Durchführung geht man in der Regel so vor, dass Isobuten zunächst durch Abkühlen auf unter -7°C kondensiert wird und anschließend mit der Verbindung (III) in Gegenwart des Säurekatalysators und des Lösungsmittels bei der gewünschten Reaktionstemperatur umgesetzt wird. Bei der Durchführung unter Druck braucht Isobuten nicht kondensiert werden. Der Druck beträgt vorzugsweise 1,5 bis 20 bar, besonders bevorzugt 2 bis 20 bar.

Die geschützte Verbindung (IV) wird anschließend nach üblichen Verfahren isoliert und gegebenenfalls gereinigt. Beispielsweise kann die Reaktionslösung, vorzugsweise nach dem Entfernen von überschüssigem Isobuten, zunächst neutralisiert werden, z.B. durch Rühren mit einer basischen wässrigen Lösung und/oder durch Extraktion mit Wasser oder einer wässrigen basischen Lösung, Trocknen der organischen Lösungsmittelphase und Entfernen des Lösungsmittels. Gewünschtenfalls kann das Produkt (IV) weiter aufgereinigt werden, beispielsweise chromatographisch oder extraktiv.

Anschließend wird das geschützte Produkt (IV) einer reduktiven Aminierung zum racemischen Amin (II) unterworfen. Geeignete Reaktionsbedingungen für reduktive Aminierungen sind beispielsweise in Jerry March, Advanced Organic Chemistry, 3. Aufl., John Wiley and Sons, S. 798 ff. beschrieben, worauf hiermit in vollem Umfang Bezug genommen wird. Als Aminierungs- und Reduktionskomponenten werden vorzugsweise Ammoniak und Wasserstoff eingesetzt. Möglich ist jedoch auch die Umsetzung mit anderen Reduktionsmitteln, wie Ammoniak in Kombination mit Zink und HCl, Natriumcyanoborhydrid (NaBH₃CN), Natriumborhydrid, Eisenpentacarbonyl mit alkoholischer KOH oder Selenophenol (PhSeH). Bevorzugt wird jedoch die Verwendung von Ammoniak in Kombination mit Wasserstoff. In diesem Fall erfolgt die Umsetzung in der Regel in Gegenwart eines Hydrierungskatalysators, wobei der Hydrierungskatalysator sowohl homogen als auch heterogen sein kann.

Handelt es sich bei der in Schritt (a) eingeführten Schutzgruppe um eine Benzylgruppe, so wird der Hydrierkatalysator vorzugsweise so ausgewählt, dass er möglichst milde Reaktionsbedingungen erlaubt und so die hydrogenolytische Abspaltung der Benzylschutzgruppe im Verlauf der reduktiven Aminierung vermieden wird. Alternativ wird in Gegenwart einer Benzylschutzgruppe anstelle von Wasserstoff ein anderes Reduktionsmittel, z.B. Natriumcyanoborhydrid, verwendet.

Als Hydrierungskatalysatoren sind unter der obigen Maßgabe alle Katalysatoren des Standes der Technik geeignet, welche die reduktive Amininierung von Ketonen und Aldehyden zu den entsprechenden Aminen katalysieren. Vorzugsweise enthalten die Hydrierungskatalysatoren wenigstens ein Metall der Gruppe VIII.

Besonders geeignete Metalle der Gruppe VIII sind ausgewählt unter Ruthenium, Cobalt, Rhodium, Nickel, Palladium und Platin.

Die Metalle können auch als Gemische eingesetzt werden. Außerdem können die Katalysatoren neben den Metallen der Gruppe VIII auch geringe Mengen weiterer Metalle, beispielsweise Metalle der Gruppe Vlla, insbesondere Rhenium, oder Metalle der Gruppe Ib, d. h. Kupfer, Silber oder Gold, enthalten. Besonders bevorzugte Metalle der Gruppe VIII sind Ruthenium, Nickel, Palladium und Platin, insbesondere Ruthenium, Nickel und Palladium, und stärker bevorzugt Ruthenium und Nickel. Speziell enthält der Katalysator Nickel als katalytisch aktive Spezies.

Wird ein Heterogenkatalysator eingesetzt, so liegt dieser geeigneter Weise in feinverteilter Form vor. Die feinverteilte Form wird beispielsweise folgendermaßen erreicht:
a) Schwarzkatalysator: Das Metall wird kurz vor der Verwendung als Katalysator aus der Lösung eines seiner Salze reduktiv abgeschieden.
b) Adams-Katalysator: Die Metalloxide, insbesondere die Oxide von Platin und Palladium, werden in situ durch den zur Hydrierung eingesetzten Wasserstoff reduziert.
c) Skelett- oder Raney-Katalysator: Der Katalysator wird als "Metallschwamm" aus einer binären Legierung des Metalls (insbesondere Nickel oder Cobalt) mit Aluminium oder Silicium durch Herauslösen eines Partners mit Säure oder Lauge hergestellt. Reste des ursprünglichen Legierungspartners wirken oft synergistisch.
d) Trägerkatalysator: Schwarzkatalysatoren lassen sich auch auf der Oberfläche einer Trägersubstanz niederschlagen. Geeignete Träger und Trägermaterialien sind nachfolgend beschrieben.

Solche Heterogenkatalysatoren sind in allgemeiner Form beispielsweise im Organikum, 17. Auflage, VEB Deutscher Verlag der Wissenschaften, Berlin, 1988, S. 288 beschrieben.

Die Träger können aus metallischem oder nichtmetallischem, porösem oder nichtporösem Material bestehen.

Geeignete metallische Materialien sind beispielsweise hochlegierte Edelstähle. Geeignete nichtmetallische Materialien sind beispielsweise mineralische Werkstoffe, z.B. natürliche und synthetische Mineralien, Gläser oder Keramiken, Kunststoffe, z.B. künstliche oder natürliche Polymere, oder eine Kombination aus beiden.

Bevorzugte Trägermaterialien sind Kohle, insbesondere Aktivkohle, Siliciumdioxid, insbesondere amorphes Siliciumdioxid, Aluminiumoxid, und außerdem die Sulfate und Carbonate der Erdalkalimetalle, Calciumcarbonat, Calciumsulfat, Magnesiumcarbonat, Magnesiumsulfat, Bariumcarbonat und Bariumsulfat.

Der Katalysator kann durch übliche Verfahren auf den Träger aufgebracht werden, z.B. durch Tränken, Benetzen oder Besprühen des Trägers mit einer Lösung, die den Katalysator oder eine geeignete Vorstufe davon enthält.

Geeignete Träger und Verfahren zum Aufbringen des Katalysators auf diese sind beispielsweise in der DE-A-10128242 beschrieben, auf die hiermit in vollem Umfang Bezug genommen wird.

Auch homogene Hydrierungskatalysatoren können in das erfindungsgemäße Verfahren eingesetzt werden. Beispiele hierfür sind die Nickelkatalysatoren, die in der EP-A-0668257 beschrieben sind. Nachteilig bei einer Verwendung von Homogenkatalysatoren ist jedoch ihre Herstellungskosten und auch die Tatsache, dass sie in der Regel nicht regenerierbar sind.

Daher werden im erfindungsgemäßen Verfahren vorzugsweise heterogene Hydrierungskatalysatoren eingesetzt.

Besonders bevorzugt wird das Metall in geträgerter Form oder als Metallschwamm eingesetzt. Beispiele für geträgerte Katalysatoren sind insbesondere Palladium, Nickel oder Ruthenium auf Kohle, insbesondere Aktivkohle, Siliciumdioxid, insbesondere auf amorphem Siliciumdioxid, Bariumcarbonat, Calciumcarbonat, Magnesiumcarbonat oder Aluminiumoxid, wobei die Träger in den oben beschriebenen Formen vorliegen können. Bevorzugte Trägerformen sind die oben beschriebenen Formkörper.

Die metallischen Katalysatoren können auch in Form ihrer Oxide, insbesondere Palladiumoxid, Platinoxid oder Nickeloxid, eingesetzt werden, die dann unter den Hydrierbedingungen zu den entsprechenden Metallen reduziert werden.

Als Metallschwamm wird insbesondere Raney-Nickel eingesetzt.

Speziell verwendet man im erfindungsgemäßen Verfahren Raney-Nickel als Hydrierungskatalysator.

Die einzusetzende Katalysatormenge hängt unter anderem vom jeweiligen katalytisch aktiven Metall und von dessen Einsatzform ab und kann vom Fachmann im Einzelfall bestimmt werden.

Die reduktive Aminierung erfolgt bei einer Temperatur von vorzugsweise 20 bis 200°C, besonders bevorzugt von 50 bis 150°C und insbesondere von 70 bis 120°C.

Der Reaktionsdruck der reduktiven Aminierung liegt vorzugsweise im Bereich von 2 bis 300 bar, besonders bevorzugt von 50 bis 150 bar.

Sowohl Reaktionsdruck als auch Reaktionstemperatur hängen unter anderem von der Aktivität und Menge des eingesetzten Hydrierungskatalysators ab und können im Einzelfall vom Fachmann bestimmt werden.

Die reduktive Aminierung des Schritts (b) kann in einem geeigneten Lösungsmittel erfolgen. Geeignete Lösungsmittel sind mit Ausnahme der halogenierten Kohlenwasserstoffe die vorstehend zu Schritt (i) genannten. Vorzugsweise erfolgt die Reaktion jedoch in Abwesenheit eines organischen Lösungsmittels. Statt dessen dient in einer bevorzugten Ausführungsform kondensierter Ammoniak, der im Überschuss eingesetzt wird, als Lösungsmittel.

Nach beendeter Reaktion wird nach dem Entspannen des Reaktionsgefäßes in der Regel überschüssiger Ammoniak abgedampft und der Katalysator entfernt. Der heterogene Katalysator wird vorzugsweise durch Filtration oder durch Sedimentation und Entfernung der oberen, produkthaltigen Phase abgetrennt. Auch andere Abtrennungsverfahren zur Entfernung von Feststoffen aus Lösungen, wie beispielsweise Zentrifugieren, sind zur Entfernung der heterogenen Katalysatoren geeignet. Die Entfernung homogener Katalysatoren erfolgt durch übliche Verfahren zur Trennung von gleichphasigen Gemischen, beispielsweise durch chromatographische Methoden. Gegebenenfalls kann es je nach Katalysatortyp erforderlich sein, diesen vor der Entfernung zu deaktivieren. Dies kann durch übliche Verfahren erfolgen, beispielsweise durch Waschen der Reaktionslösung mit protischen Lösungsmitteln, z.B. mit Wasser oder mit C₁-C₃-Alkanolen, wie Methanol, Ethanol, Propanol oder Isopropanol, die erforderlichenfalls basisch oder sauer eingestellt sind. Falls ein Lösungsmittel bei der Umsetzung verwendet wurde, wird dieses in der Regel ebenfalls entfernt, was durch übliche Verfahren, beispielsweise destillativ, insbesondere unter verringertem Druck, erfolgen kann.

Das Reaktionsprodukt kann durch übliche Verfahren aufgereinigt werden, beispielsweise durch Destillation, Sublimierung, Extraktion oder Chromatographie.

Bei der Durchführung der Reaktion in umgekehrter Reihenfolge, d.h. zuerst reduktive Aminierung und erst anschließend Schutzgruppeneinführung, gilt für den Schritt der reduktiven Aminierung das zuvor Gesagte, wobei jedoch das Problem der Abspaltung bestimmter Schutzgruppen unter den Aminierungsbedingungen nicht existiert. Allerdings kann bei dieser Reaktionsfolge keine Benzylschutzgruppe verwendet werden, da diese bevorzugt mit der erhaltenen Aminogruppe reagieren würde und ein gebildetes Benzylamin auch schwerer zu entschützen ist als ein Benzylether. Hier wird vorzugsweise ein tert-Butylether als Schutzgruppe eingesetzt. Zur Einführung der Schutzgruppe gilt das zuvor Gesagte, wobei bei der Umsetzung unter Säurekatalyse beachtet werden muss, dass die Säure in mehr als äquimolarer Menge, in Bezug auf die zu schützende Verbindung, eingesetzt werden muss, da sie von der Aminofunktion zumindest teilweise gebunden wird.

Bevorzugt ist die Herstellung der Verbindung (II) in der Reihenfolge der Schritte (a) und anschließend (b).

Auch in den Verbindungen (III), (IV) und (VI) steht n vorzugsweise für 0 oder 2 und insbesondere für 0. m steht vorzugsweise für 0 oder 1 und insbesondere für 0. Auch in diesen Verbindungen kann die Hydroxyfunktion (in Verbindungen (III) und (VI)) bzw. die geschützte Hydroxyfunktion PO (in Verbindung (IV)) in ortho-, meta- oder para-Position zur Alkylcarbonylgruppe bzw. Aminoalkylgruppe stehen. Vorzugsweise steht die Hydroxyfunktion bzw. die geschützte Hydroxyfunktion PO in meta- oder para-Position und insbesondere in para-Position zur Alkylcarbonylgruppe bzw. Aminoalkylgruppe.

Schließlich ist Gegenstand der Erfindung ein \/erfahren zur Herstellung von optisch aktiven Verbindungen der Formeln I-S und/oder I-R 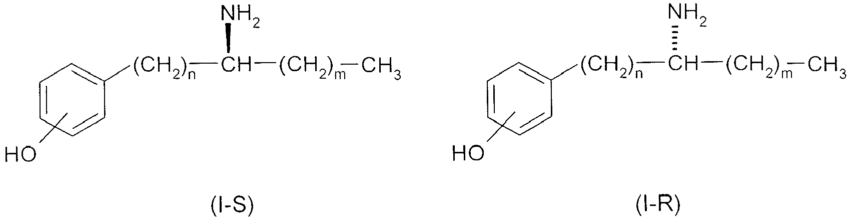 worin n und m wie vorstehend definiert sind, bei dem man
(a) eine Verbindung der Formel (III) 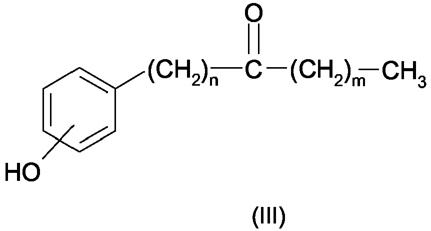 unter Erhalt der Verbindung (IV) 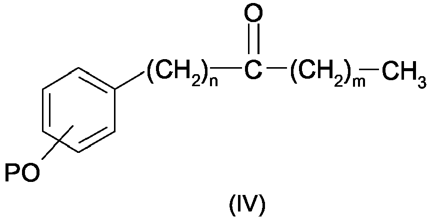 worin P wie vorstehend definiert ist,
   an der OH-Gruppe schützt;
(b) die Verbindung (IV) einer reduktiven Aminierung unterwirft, wobei man ein Enantiomerengemisch der Formel (II) 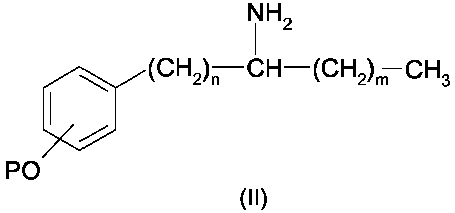 erhält;
(c) das Enantiomerengemisch der Formel (II) mit einem Acylierungsmittel in Gegenwart einer Hydrolase umsetzt, wobei man ein Gemisch erhält, in welchem ein Enantiomer im Wesentlichen in der acylierten Form vorliegt und das andere Enantiomer im Wesentlichen in der nicht acylierten Form vorliegt;
(d) das nicht acylierte Enantiomer der Verbindung (II) aus dem in Schritt (i) erhaltenen Gemisch abtrennt;
(e) dass in Schritt (ii) gewonnene nicht acylierte Enantiomer der Verbindung (II) zum Amin (I-S) oder (I-R) entschützt; und
(f) gewünschtenfalls das in Schritt (i) erhaltene im Wesentlichen acylierte Enantiomer der Verbindung (II) zum entsprechenden nicht acylierten Enantiomer des Amins (II) hydrolysiert und anschließend zum Amin (I-R) oder (I-S) entschützt.

Die vorstehend gemachten Ausführungen zu geeigneten und bevorzugten Ausgestaltungen der Verbindungen und des Verfahrens gelten hier entsprechend.

Durch das erfindungsgemäße Verfahren, insbesondere in derjenigen Ausführungsform, welche die Schritte (a) und (b) umfasst, erhält man die Verbindungen (I-S) und/oder (I-R) in hohen Ausbeuten und mit einer sehr großen Enantiomerenreinheit.

Die vorliegende Erfindung wird durch die nachfolgenden, nicht einschränkenden Beispiele veranschaulicht.

### Beispiele

### 1. Synthese von (S)-4-(1-Aminoethyl)phenol und (R)-4-(1-Aminoethyl)phenol

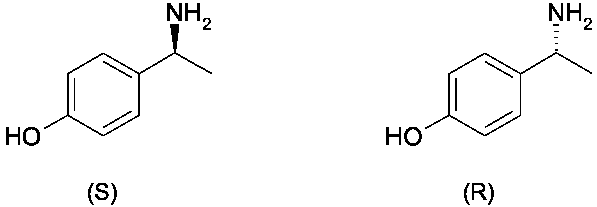

### 1.1 Einführung der Schutzgruppe: Umsetzung von 4-Hydroxyacetophenon zu 4-tert-Butoxyacetophenon

In einem Dreihalskolben mit Trockeneiskühler wurde p-Hydroxyacetophenon (60 g, 0,44 mol) in Methylenchlorid (800 ml) mit 3 Tropfen Trifluormethansulfonsäure versetzt. Zu dem Reaktionsgemisch gab man kondensiertes Isobuten (123 g, 2,2 mol) hinzu, wobei eine Trübung auftrat und ein feinkristalliner Niederschlag ausfiel. Das leicht violettfarbene Gemisch wurde über Nacht mit aufgesetztem Trockeneiskühler gerührt, wobei Trockeneis und überschüssiges Isobuten abdampften. Am nächsten Tag wurde das Gemisch unter Rühren in halbkonzentrierte Natriumcarbonatlösung (600 ml) gegeben. Nach der Phasentrennung wurde die organische Phase so oft mit halbgesättigter Natriumcarbonatlösung gewaschen, bis die wässrige Phase farblos blieb. Die organische Phase wurde über Natriumsulfat getrocknet und das Lösungsmittel wurde unter verringertem Druck entfernt. Man erhielt 66 g (78 % der Theorie) 4-tert-Butoxy-acetophenon als farbloses Öl, welches laut GC-Analytik eine Reinheit von 99,9% aufwies.
¹H-NMR (400 MHz; CDCl₃): δ = 1,40 (s, 9H); 2,58 (s, 3H); 7,05 und 7,90 (AA',BB'-System; J_{AB} = 10,5 Hz, 4H).

### 1.2 Reduktive Aminierung: Synthese von 1-(4-tert-Butoxyphenyl)ethylamin

Ein Gemisch aus 4-tert-Butoxyacetophenon aus Beispiel 1.1 (44 g, 0,23 mol), das mit 5 Tropfen konzentrierter Ammoniaklösung stabilisiert worden war, und 1 g Raney-Nickel wurden mit 3 Tropfen Eisessig versetzt und in einem Autoklaven in flüssigem Ammoniak (100 ml) gelöst. Das Reaktionsgemisch wurde auf 80 °C erhitzt und Wasserstoff wurde bis zu einem Innendruck von 100 bar eingeführt. Anschließend rührte man bei 100 °C, bis kein weiterer Wasserstoff mehr aufgenommen wurde (10-20 h). Der Autoklav wurde entspannt, der Rückstand wurde nach dem Abdampfen des Ammoniaks in Methanol (200 ml) aufgenommen und der Katalysator wurde über Kieselgur abfiltriert. Das Filtrat wurde eingeengt und der verbliebene Destillationsrückstand wurde im Ölpumpenvakuum bei 85 °C umkondensiert. Man erhielt 40 g (90 % der Theorie) rohes Amin, das laut GC-Analyse eine Reinheit von 95,6 % aufwies. Die Kondensate aus mehreren Ansätzen wurden gesammelt einer Vakuumdestillation unterworfen. Man erhielt 1-(4-tert-Butoxyphenyl)ethylamin in einer Ausbeute von 75 % der Theorie als Reinprodukt.
Siedepunkt: 94 - 97 °C (0,4 mm)
¹H-NMR (400 MHz; CDCl₃): δ = 1,65 (s, 9H); 1,80 (d, J = 7Hz, 3H); 4,20 (q, J = 7Hz, 1 H); 4,40 (s, breit, 2H); 6,80 und 7,50 (AA',BB'-System; J_{AB} = 10,5 Hz, 4H).

### 1.3 Racematspaltung

1-(4-tert-Butoxyphenyl)ethylamin (246 g, 1,28 mol) wurde mit Methoxyessigsäureisopropylester (254 g, 1,92 mol) und Novozyme® 435 (2,5 g) versetzt und bei Raumtemperatur gerührt. Nach einer Stunde fiel ein weißer, feinkristalliner Rückstand aus. Nach dem Erwärmen des Gemischs auf 35 °C rührte man es über Nacht bei dieser Temperatur. Die anschließende Analyse der optischen Reinheit zeigte, dass das S-Enantiomer von 1-(4-tert-Butoxyphenyl)ethylamin enantiomerenrein war. Das acylierte R-Enantiomer wies eine optische Reinheit von 98,9 % auf. Das Gemisch wurde über Kieselgur filtriert, mit Toluol (200 ml) gewaschen und das Filtrat wurde am Rotationsverdampfer bei 40 °C eingeengt. Der Rückstand wurde in Toluol (400 ml) aufgenommen und tropfenweise mit 38%iger Schwefelsäure (82 g, 0,31 mol) versetzt. Es bildete sich ein weißer dicker Niederschlag, der mit Toluol (500 ml) verdünnt wurde. Der Feststoff wurde abgesaugt, mit Toluol (300 ml) gewaschen und über Nacht im Vakuumtrockenschrank bei 40 °C getrocknet. Man erhielt 144,4 g (96 %) des Sulfatsalzes von (S)-1-(4-tert-Butoxyphenyl)ethylamin.

Zur Isolierung des acylierten R-Enantiomers von 1-(4-tert-Butoxyphenyl)ethylamin wurde die Wasserphase der vereinigten Filtrate abgetrennt und die organische Phase wurde eingeengt, bis ein weißer schmieriger Feststoff (256 g) zurückblieb. Der Feststoff wurde aufgeschmolzen und im Ölpumpenvakuum bei einer Badtemperatur von 70 °C von allen flüchtigen Bestandteilen befreit. Man erhielt 180 g (quantitative Ausbeute) rohes (R)-N-1-(4-tert-Butoxyphenyl)ethylmethoxyacetamid, das noch mit Methoxyessigsäureisopropylester (ca. 5 %) verunreinigt war. Die optische Reinheit betrug 98,9 % ee.
¹H-NMR von (R)-N-1-(4-tert-Butoxyphenyl)ethylmethoxyacetamid
¹H-NMR (400 MHz; CDCl₃): δ = 1,30 (s, 9H); 1,50 (d, J = 7Hz, 3H); 3,40 (s, 3H); 3,87 und 3,95 (AB-System; J_{AB}= 16 Hz, 2H); 5,15 (dq, J = 7Hz und 9 Hz, 1H); 6,70 (s, breit, 1H); 6,95 und 7,20 (AA',BB'-System; J_{AB} = 10,5 Hz, 4H).

### 1.4 Amidhydrolyse: Synthese von (R)-1-(4-tert-Butoxyphenyl)ethylamin

(R)-N-1-(4-tert-Butoxyphenyl)ethylmethoxyacetamid aus Beispiel 1.3 (180 g mit einer Reinheit von 94 %; entspricht 0,64 mol Reinsubstanz) wurde unter Rühren mit Triethanolamin (20 ml) gemischt und anschließend mit 50%iger NaOH (70,8 g, 0,89 mol) versetzt. Dann rührte man bei einer Badtemperatur von 140 °C noch sieben Stunden, wonach sich laut GC sich das gesamte Amid umgesetzt hatte. Die Mischung wurde mit Wasser (100 ml) verdünnt, abgekühlt und mit tert-Butylmethylether (3 x 100 ml) extrahiert. Die vereinten Extrakte wurden mit gesättigter Kochsalzlösung (50 ml) gewaschen und über Na₂SO₄ getrocknet. Nach dem Entfernen des Lösungsmittels am Rotationsverdampfer erhielt man 123,7 g (73 %) (R)-1-(4-tert-Butoxyphenyl)ethylamin in Form eines schwach braunen Öls. Die chemische Reinheit betrug 99,4 %, die optische Reinheit 98,9 % ee.
Das ¹H-NMR-Spektrum ist mit dem in Beispiel 1.2 angegebenen identisch.

### 1.5 Abspaltung der Schutzgruppe: Synthese von (R)-4-(1-Aminoethyl)phenol

(R)-1-(4-tert-Butoxyphenyl)ethylamin (89,4 g, 0,463 mol) wurde in 10%iger Salzsäure (200 ml) gelöst und auf 85 °C erhitzt. Bei ca. 80 °C trat eine heftige Gasentwicklung ein. Man rührte noch drei Stunden bei 85°C nach, wonach die Gasentwicklung zum Erliegen gekommen war. Man ließ die schwach gelbe Mischung auf Raumtemperatur abkühlen und tropfte dann 20%ige NaOH bis zu einem pH-Wert von 10,2 zu. Über Nacht fiel das entschützte Phenol in Form eines schwach gelben Pulvers aus. Dieses wurde abgesaugt und über Nacht im Trockenschrank getrocknet. Zur Reinigung wurde das Rohprodukt mit tert-Butylmethylether (200 ml) digeriert. Die Suspension wurde filtriert und der Filterrückstand wurde erneut im Vakuum getrocknet. Man erhielt 51,3 g (63 %) (R)-4-(1-Aminoethyl)phenol in Form eines schwach gelben Pulvers.
Fp: 121 °C.
Drehwert [α]_{D} = 27,5° (c = 1 in Methanol).
¹H-NMR (400 MHz; DMSO-d₆): δ = 1,15 (d, J = 7Hz, 3H); 1,75 (s, breit, 2H); 3,90 (q, J = 7 Hz, 1H); 6,65 und 7,15 (AA',BB'-System; J_{AB} =10,5 Hz, 4H); 9,20 (s, breit, 1H).

### 1.6 Abspaltung der Schutzgruppe: Synthese von (S)-4-(1-Aminoethyl)phenol

(S)-1-(4-tert-Butoxyphenyl)ethylammoniumsulfat aus Beispiel 1.3 (144 g, 0,61 mol) wurde in Wasser (500 ml) suspendiert. Die Suspension wurde durch Zugabe von konz. H₂SO₄ (3 ml) auf einen pH-Wert von 1 eingestellt und auf 85 °C erhitzt. Es trat eine leichte Gasentwicklung ein. Nach drei Stunden war das gesamte Salz in Lösung gegangen und die Gasentwicklung zum Erliegen gekommen. Man ließ abkühlen und versetzte die Mischung mit 20%iger NaOH, bis der pH-Wert 10,2 betrug. Am nächsten Tag wurde der über Nacht ausgefallene Feststoff abgesaugt und im Vakuumtrockenschrank getrocknet. Da das Rohprodukt noch unlösliches Natriumsulfat enthielt, wurde es in Methanol (1500 ml) aufgenommen und das unlösliche Na₂SO₄ abfiltriert. Das Filtrat wurde eingeengt und der Rückstand wurde mit tert-Butylmethylether (200 ml) digeriert. Nach dem Absaugen und Trocknen des Feststoffs im Vakuum erhielt man 68 g (58 % der Theorie) (S)-4-(1-Aminoethyl)phenol in Form eines schwach gelben Pulvers.
Fp: 120 °C.
Drehwert [α]_{D} = -28° (c = 1 in Methanol).
Das ¹H-NMR-Spektrum des Produkts ist mit dem in Beispiel 1.5 angegebenen identisch.

### 2. Synthese von (S)-3-(1-Aminoethyl)phenol und (R)-3-(1-Aminoethyl)phenol

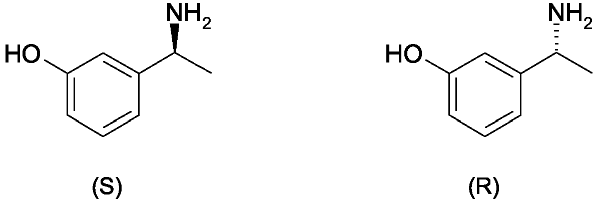

### 2.1 Einführung der Schutzgruppe: Umsetzung von 3-Hydroxyacetophenon zu 3-tert-Butoxyacetophenon

In einem Dreihalskolben mit Trockeneiskühler wurde meta-Hydroxyacetophenon (50 g, 0,37 mol) in Methylenchlorid (1 l) mit 3 Tropfen Trifluormethansulfonsäure versetzt. Zu dem Reaktionsgemisch gab man kondensiertes Isobuten (103 g, 1,84 mol) hinzu, wobei eine Trübung auftrat und ein feinkristalliner Niederschlag ausfiel. Das leicht gelbe Gemisch wurde über Nacht (16 h) mit aufgesetztem Trockeneiskühler gerührt, wobei Trockeneis und überschüssiges Isobuten abdampften. Am nächsten Tag wurde das orangefarbene Gemisch unter Rühren in halbkonzentrierte Natriumcarbonatlösung (500 ml) gegeben. Nach der Phasentrennung wurde die organische Phase 4 Mal mit je 150 ml halbgesättigter Natriumcarbonatlösung gewaschen, bis die wässrige Phase farblos blieb. Die organische Phase wurde über Natriumsulfat getrocknet und das Lösungsmittel wurde unter verringertem Druck entfernt. Man erhielt 60 g (87 % der Theorie) 3-tert-Butoxyacetophenon als farbloses Öl, welches laut GC-Analytik eine Reinheit von 95% aufwies.
¹H-NMR (400 MHz; CDCl₃): δ = 1,40 (s, 9H); 2,58 (s, 3H); 7,20 (m, 1H); 7,35 (dd; J= 10 und 12 Hz, 1H); 7,58 (m, 1H), 7,68 (m, 1H).

### 2.2 Reduktive Aminierung: Synthese von 1-(3-tert-Butoxyphenyl)ethylamin

Ein Gemisch aus 3-tert-Butoxyacetophenon aus Beispiel 2.1 (60 g, 0,32 mol), das mit 5 Tropfen konzentrierter Ammoniaklösung stabilisiert worden war, und 1 g Raney-Nickel wurden mit 3 Tropfen Eisessig versetzt und in einem Autoklaven in flüssigem Ammoniak (100 ml) gelöst. Das Reaktionsgemisch wurde auf 80 °C erhitzt und Wasserstoff wurde bis zu einem Innendruck von 100 bar eingeführt. Anschließend rührte man bei 100 °C, bis kein weiterer Wasserstoff mehr aufgenommen wurde (10-20 h). Der Autoklav wurde entspannt, der Rückstand wurde nach dem Abdampfen des Ammoniaks in Methanol (200 ml) aufgenommen und der Katalysator wurde über Kieselgur abfiltriert. Das Filtrat wurde eingeengt und der verbliebene Destillationsrückstand wurde im Ölpumpenvakuum bei 85 °C umkondensiert. Man erhielt 57,4 g (93 % der Theorie) rohes Amin, das laut GC-Analyse eine Reinheit von 91 % aufwies. Die Kondensate aus mehreren Ansätzen wurden gesammelt einer Vakuumdestillation unterworfen. Man erhielt 1-(3-tert-Butoxyphenyl)ethylamin in einer Ausbeute von 72 % der Theorie als Reinprodukt.
Siedepunkt: 89 - 90 °C (0,4 mm)
¹H-NMR (400 MHz; CDCl₃): δ = 1,35 (s, 9H); 1,38 (d, J = 7Hz, 3H); 1,58 (s, breit, 2H); 4,10 (m, 1H); 6,88 (m, 1H); 6,95 (m, 1H); 7,05 (m, 1H); 7,20 (dd; J = 10 und 12 Hz, 1 H).

### 2.3 Racematspaltung

1-(3-tert-Butoxyphenyl)ethylamin (86 g, 0,45 mol) wurde mit Methoxyessigsäureisopropylester (129 g, 0,98 mol) und Novozyme® 435 (0,9 g) versetzt und bei Raumtemperatur 16 h gerührt. Die anschließende Analyse der optischen Reinheit zeigte, dass das S-Enantiomer von 1-(3-tert-Butoxyphenyl)ethylamin enantiomerenrein war. Das acylierte R-Enantiomer wies eine optische Reinheit von 99,1 % auf. Das Gemisch wurde über Kieselgur filtriert, mit Toluol (200 ml) gewaschen und das Filtrat wurde am Rotationsverdampfer bei 40 °C eingeengt. Der Rückstand wurde in Toluol (200 ml) aufgenommen und tropfenweise mit 37%iger Schwefelsäure (29,5 g, 0,12 mol) versetzt. Es bildete sich ein schmieriger, nicht filtrierbarer Niederschlag, der in Wasser (250 ml) gelöst wurde. Die wässrige Lösung wurde 3 mal mit je 50 ml Toluol gewaschen. Die vereinten organischen Phasen wurden 1 mal mit 50 ml Wasser gewaschen und anschließend über Natriumsulfat getrocknet. Nach dem Entfernen des Lösungsmittels wurde der Rückstand im Olpumpenvakuum bei 0,5 bar und 85 °C von weiteren flüchtigen Bestandteilen befreit. Man erhielt 70 g rohes (R)-N-1-(3-tert-Butoxyphenyl)ethylmethoxy-acetamid, dessen chemische Reinheit laut GC-Analytik 93 % betrug. Die optische Reinheit betrug 99 % ee.

Zur Isolierung von (S)-1-(3-tert-Butoxyphenyl)ethylamin wurden die vereinten wässrigen Phasen durch Zugabe von festem Natriumhydroxid auf pH 14 eingestellt. Anschließend gab man Toluol (100 ml) zu, trennte die Phasen und extrahierte die wässrige Phase noch 3 Mal mit je 50 ml Toluol. Die vereinten organischen Phasen wurden über Natriumsulfat getrocknet und unter verringertem Druck vom Lösungsmittel befreit. Man erhielt 40,9 g (94 %) reines (S)-1-(3-tert-Butoxyphenyl)ethylamin als farbloses Öl mit einer Enantiomerenreinheit von über 99,9% ee.
¹H-NMR von (R)-N-1-(3-tert-Butoxyphenyl)ethylmethoxyacetamid:
¹H-NMR (400 MHz; CDCl₃): δ = 1,35 (s, 9H); 1,52 (d, J = 7 Hz, 3H); 3,40 (s, 3H); 3,90 und 3,95 (AB-System; J_{AB} = 16 Hz, 2H); 5,15 (dq, J = 7 Hz und 9 Hz, 1 H); 6,80 (s, breit, 1H); 6,92 (m, 1H); 6,95 (m, 1H); 7,05 (m, 1H); 7,25 (dd; J = 10 und 12 Hz, 1 H).

### 2.4 Amidhydrolyse: Synthese von (R)-1-(3-tert-Butoxyphenyl)ethylamin

(R)-N-1-(3-tert-Butoxyphenyl)ethylmethoxyacetamid aus Beispiel 2.3 (63,5 g mit einer Reinheit von 93 %; entspricht 0,22 mol Reinsubstanz) wurde unter Rühren mit Triethanolamin (6 ml) gemischt und anschließend mit 50%iger NaOH (23,2 g, 0,29 mol) versetzt. Dann rührte man bei einer Badtemperatur von 140 °C noch 16 Stunden, wonach sich laut GC-Analyse das gesamte Amid umgesetzt hatte. Die Mischung wurde mit Wasser (100 ml) verdünnt, abgekühlt und mit tert-Butylmethylether (3 x 100 ml) extrahiert. Die vereinten Extrakte wurden mit gesättigter Kochsalzlösung (50 ml) gewaschen und über Na₂SO₄ getrocknet. Nach dem Entfernen des Lösungsmittels am Rotationsverdampfer erhielt man 41 g (92 %) (R)-1-(3-tert-Butoxyphenyl)ethylamin in Form eines farblosen Öls. Die chemische Reinheit betrug 99,4 %, die optische Reinheit 98,9 % ee.
Das ¹H-NMR-Spektrum ist mit dem in Beispiel 2.2 angegebenen identisch.

### 2.5 Abspaltung der Schutzgruppe: Synthese von (R)-3-(1-Aminoethyl)phenol

(R)-1-(3-tert-Butoxyphenyl)ethylamin (5 g, 25,9 mmol) wurde in Wasser (10 ml) suspendiert und mit 38%iger Schwefelsäure bis zu einem pH von 1 versetzt. Das Gemisch wurde auf 85 °C erhitzt und noch drei Stunden bei dieser Temperatur gerührt, wonach die Gasentwicklung zum Erliegen gekommen war. Man ließ die farblose Mischung auf Raumtemperatur abkühlen und tropfte dann 20%ige NaOH bis zu einem pH-Wert von 9,1 zu, worauf das entschützte Phenol in Form eines weißen Pulvers ausfiel. Dieses wurde abgesaugt und über Nacht im Trockenschrank getrocknet. Man erhielt 2,6 g (73 %) (R)-3-(1-Aminoethyl)phenol in Form eines schwach gelben Pulvers.
Fp: 160 °C.
Drehwert [α]_{D} = 22,5° (c = 1 in Methanol).
¹H-NMR (400 MHz; CDCl₃): δ = 1,35 (d, J = 7Hz, 3H); 2,15 (s, breit, 3H); 4,05 (q, J = 7 Hz, 1 H); 6,75 (m, 1 H); 6,50 (m, 2H); 7,15 (m, 1 H).

### 2.6 Abspaltung der Schutzgruppe: Synthese von (S)-3-(1-Aminoethyl)phenol

Die Schutzgruppenabspaltung an (S)-1-(3-tert-Butoxyphenyl)ethylamin aus Beispiel 2.3 erfolgte analog zu Beispiel 2.5. Man erhielt reines (S)-3-(1-Aminoethyl)phenol in einer Ausbeute von 78 % der Theorie und einer Enantiomerenreinheit von über 99,9 % ee.
Drehwert [α]_{D} = 22,4° (c = 1 in Methanol).
Das ¹H-NMR-Spektrum ist mit dem in Beispiel 2.5 angegebenen identisch.

## Patentansprüche

1. Verfahren zur Herstellung von optisch aktiven Verbindungen der Formeln I-S und/oder I-R 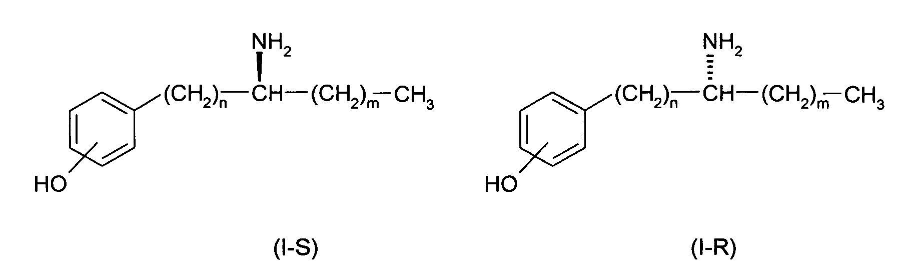 worin
n für 0, 1 oder 2 steht und
m für 0, 1 oder 2 steht,
umfassend die folgenden Schritte:
(i) Umsetzung eines Enantiomerengemischs der Formel II 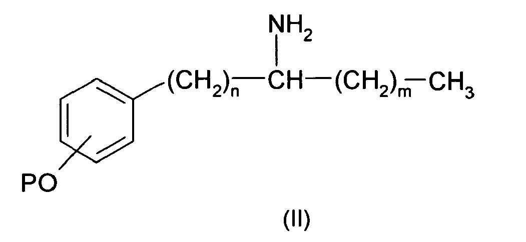 worin
P für eine Schutzgruppe steht, die ausgewählt ist unter tert-Butyl und einer am Phenylring gegebenenfalls substituierten Benzylgruppe,
mit einem Acylierungsmittel in Gegenwart einer Hydrolase, wobei man ein Gemisch erhält, in welchem ein Enantiomer der Verbindung (II) im Wesentlichen in der acylierten Form vorliegt und das andere Enantiomer der Verbindung (II) im Wesentlichen in der nicht acylierten Form vorliegt;
(ii) Abtrennung des nicht acylierten Enantiomers der Verbindung (II) aus dem in Schritt (i) erhaltenen Gemisch;
(iii) Entschützen des in Schritt (ii) gewonnenen nicht acylierten Enantiomers der Verbindung (II) zum Amin (I-S) oder (I-R); und
(iv) gewünschtenfalls Hydrolyse des in Schritt (i) erhaltenen im Wesentlichen acylierten Enantiomers der Verbindung (II) zum entsprechenden nicht acylierten Enantiomer des Amins (II) und anschließendes Entschützen zum Amin (I-R) oder (I-S);
wobei die Verbindung der Formel (II) dadurch erhalten wird, dass man
(a) eine Verbindung der Formel (III) 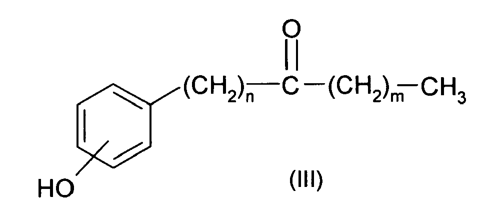 unter Erhalt der Verbindung (IV) an der OH-Gruppe schützt 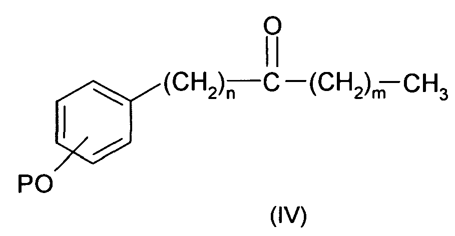 und
(b) die Verbindung (IV) einer reduktiven Aminierung unterwirft, wobei man die Verbindung der Formel (II) erhält.

2. Verfahren nach Anspruch 1, wobei es sich bei dem in Schritt (i) erhaltenen im Wesentlichen nicht acylierten Enantiomer um das S-Enantiomer der Verbindung (II) handelt und es sich bei dem bei dem im Wesentlichen acylierten Enantiomer um das R-Enantiomer der Verbindung (II) handelt.

3. Verfahren nach einem der vorhergehenden Ansprüche, wobei das Acylierungsmittel ausgewählt ist unter Estern, deren Säurekomponente in α-, β- oder γ-Stellung zum Carbonyl-Kohlenstoffatom eine Sauerstoff-, Stickstoff-, Fluor- oder Schwefel-haltige Gruppe trägt.

4. Verfahren nach einem der vorhergehenden Ansprüche, wobei die Hydrolase ausgewählt ist unter Lipasen aus Bakterien der Gattung Burkholderia oder Pseudomonas oder aus Hefen der Gattung Candida.

5. Verfahren nach Anspruch 4, wobei es sich bei der Lipase um Lipase B aus Candida antarctica handelt.

6. Verfahren nach einem der vorhergehenden Ansprüche, wobei n für 0 steht.

7. Verfahren nach einem der vorhergehenden Ansprüche, wobei P für tert-Butyl (-C(CH₃)₃) steht.

8. Verfahren nach einem der vorhergehenden Ansprüche, wobei man die Umsetzung mit dem Acylierungsmittel in Gegenwart der Hydrolase in einem nichtwässrigen Reaktionsmedium durchführt.

9. Verfahren nach einem der vorhergehenden Ansprüche, wobei man in Schritt (a) die Verbindung (III) mit Isobuten in Gegenwart einer Säure umsetzt.

## Claims

1. A process for preparing optically active compounds of the formulae I-S and/or I-R 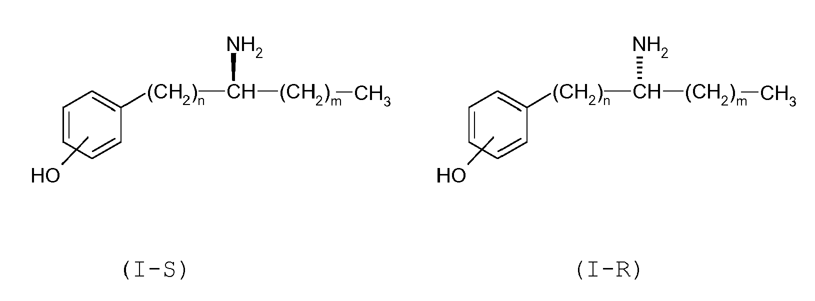 in which
n is 0, 1 or 2 and
m is 0, 1 or 2,
comprising the following steps:
(i) reacting an enantiomer mixture of the formula II 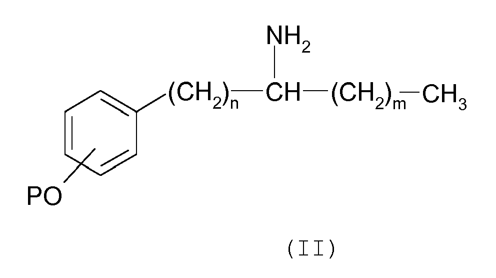 in which
P is a protecting group selected from tert-butyl and a benzyl group optionally substituted on the phenyl ring
with an acylating agent in the presence of a hydrolase to obtain a mixture in which one enantiomer of the compound (II) is present essentially in acylated form and the other enantiomer of compound (II) is present essentially in the unacylated form;
(ii) removing the unacylated enantiomer of compound (II) from the mixture obtained in step (i);
(iii) deprotecting the unacylated enantiomer of compound (II) obtained in step (ii) to give the amine (I-S) or (I-R); and
(iv) if desired hydrolyzing the essentially acylated enantiomer of compound (II) obtained in step (i) to give the corresponding unacylated enantiomer of the amine (II) and subsequently deprotecting it to give the amine (I-R) or (I-S);
wherein the compound of the formula (II) is obtained by
(a) protecting a compound of the formula (III) 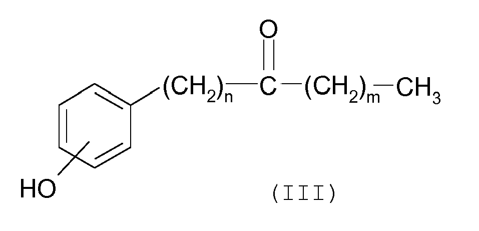 on the OH group to obtain compound (IV) 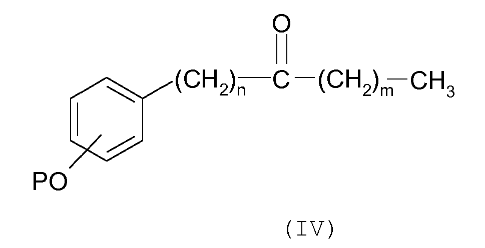 and
(b) subjecting compound (IV) to a reductive amination to obtain the compound of the formula (II).

2. The process according to claim 1, wherein the essentially unacylated enantiomer obtained in step (i) is the S enantiomer of compound (II), and the essentially acylated enantiomer is the R enantiomer of compound (II).

3. The process according to any of the preceding claims, wherein the acylating agent is selected from esters whose acid component bears an oxygen-, nitrogen-, fluorine- or sulfur-containing group in the α, β or γ position to the carbonyl carbon atom.

4. The process according to any of the preceding claims, wherein the hydrolase is selected from lipases from bacteria of the Burkholderia or Pseudomonas genera or from yeasts of the Candida genus.

5. The process according to claim 4, wherein the lipase is lipase B from Candida antarctica.

6. The process according to any of the preceding claims, wherein n is 0.

7. The process according to any of the preceding claims, wherein P is tert-butyl
(-C(CH₃)₃).

8. The process according to any of the preceding claims, wherein the reaction with the acylating agent is performed in the presence of the hydrolase in a nonaqueous reaction medium.

9. The process according to any of the preceding claims, wherein compound (III) is reacted in step (a) with isobutene in the presence of an acid.

## Revendications

1. Procédé de fabrication de composés optiquement actifs de formule I-S et/ou I-R 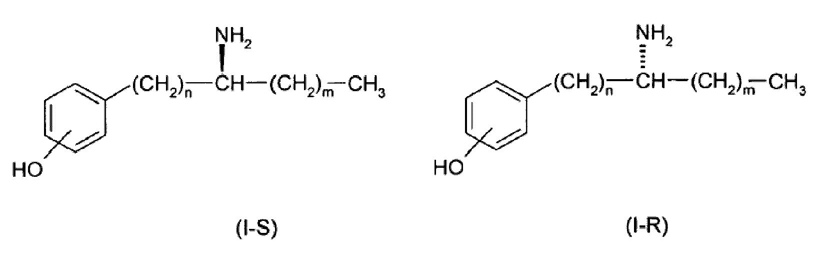 dans lesquelles
n représente 0, 1 ou 2, et
m représente 0, 1 ou 2,
comprenant les étapes suivantes :
(i) la mise en réaction d'un mélange d'énantiomères de formule II 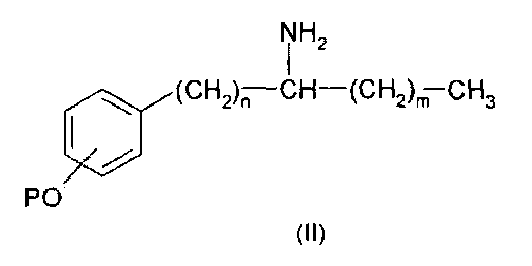 dans laquelle
P représente un groupe protecteur choisi parmi tert.-butyle et un groupe benzyle éventuellement substitué sur le cycle phényle,
avec un agent d'acylation en présence d'une hydrolase, un mélange étant obtenu, dans lequel un énantiomère du composé (II) est présent essentiellement sous la forme acylée et l'autre énantiomère du composé (II) est présent essentiellement sous la forme non acylée ;
(ii) la séparation de l'énantiomère non acylé du composé (II) du mélange obtenu à l'étape (i) ;
(iii) la déprotection de l'énantiomère non acylé du composé (II) obtenu à l'étape (ii) en l'amine (I-S) ou (I-R) ; et
(iv) si on le souhaite, l'hydrolyse de l'énantiomère essentiellement acylé du composé (II) obtenu à l'étape (i) pour former l'énantiomère non acylé correspondant de l'amine (II), puis la déprotection pour former l'amine (I-R) ou (I-S) ;
le composé de formule (II) étant obtenu par
(a) la protection d'un composé de formule (III) 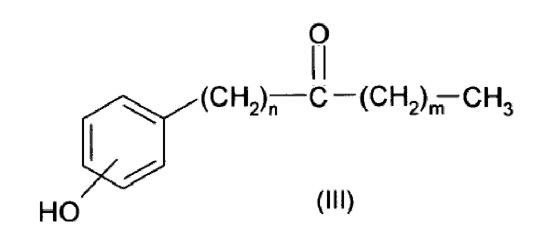 sur le groupe OH pour obtenir le composé (IV) 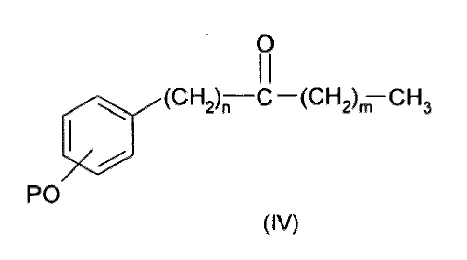 et
(b) la soumission du composé (IV) à une amination réductrice, le composé de formule (II) étant obtenu.

2. Procédé selon la revendication 1, dans lequel l'énantiomère essentiellement non acylé obtenu à l'étape (i) est l'énantiomère S du composé (II) et l'énantiomère essentiellement acylé est l'énantiomère R du composé (II).

3. Procédé selon l'une quelconque des revendications précédentes, dans lequel l'agent d'acylation est choisi parmi les esters dont le composant acide porte en position α, β ou γ par rapport à l'atome de carbone du carbonyle un groupe contenant de l'oxygène, de l'azote, du fluor ou du soufre.

4. Procédé selon l'une quelconque des revendications précédentes, dans lequel l'hydrolase est choisie parmi les lipases issues de bactéries du genre Burkholderia ou Pseudomonas ou issues de levures du genre Candida.

5. Procédé selon la revendication 4, dans lequel la lipase est la lipase B de Candida antarctica.

6. Procédé selon l'une quelconque des revendications précédentes, dans lequel n représente 0.

7. Procédé selon l'une quelconque des revendications précédentes, dans lequel P représente tert.-butyle (-C(CH₃)₃).

8. Procédé selon l'une quelconque des revendications précédentes, dans lequel la mise en réaction avec l'agent d'acylation en présence de l'hydrolase est réalisée dans un milieu réactionnel non aqueux.

9. Procédé selon l'une quelconque des revendications précédentes, dans lequel, à l'étape (a), le composé (III) est mis en réaction avec de l'isobutène en présence d'un acide.
